(19) Europäisches Patentamt
European Patent Office
Office européen des brevets

(11) EP 3 967 760 A1

(12) **EUROPEAN PATENT APPLICATION**

(43) Date of publication:
**16.03.2022 Bulletin 2022/11**

(21) Application number: **20209658.2**

(22) Date of filing: **24.11.2020**

(51) International Patent Classification (IPC):
***C12N 15/74** (2006.01)* ***C12N 15/00** (2006.01)*
***C12N 15/79** (2006.01)* ***C07K 14/47** (2006.01)*
***C12N 15/63** (2006.01)*

(52) Cooperative Patent Classification (CPC):
**C12N 15/74; C12N 15/63; C12N 15/79**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.09.2020 EP 20306020**

(71) Applicant: **Synovance**
**91058 Évry-Courcouronnes Cedex (FR)**

(72) Inventors:
- **JESTER, Brian Craig**
**91058 Évry-Courcouronnes Cedex (FR)**
- **KEPES, François**
**91058 Évry-Courcouronnes Cedex (FR)**

(74) Representative: **Flesselles, Bruno F.G.**
**BF IP**
**36 rue Jean de la Fontaine**
**75016 Paris (FR)**

(54) **METHODS FOR ENGINEERING CHROMOSOMAL ARCHITECTURE FOR GENE EXPRESSION**

(57) The invention is in the field of molecular biology and relates to methods and constructs for transformation of cells for transgene expression by creating local genomic regions and loops favorable for gene expression.

EP 3 967 760 A1

## Description

The invention is in the field of molecular biology and relates to methods and constructs for transformation of cells for transgene expression.

Synthetic biology is defined as the design and construction of new biological systems for useful purposes. Advances in this field have been hampered by the unpredictable performance of synthetic circuits and biosynthetic pathways once inserted into the genome. Recent studies have shown that a reporter gene expressed from a single promoter, flanked by transcriptional terminators, produces different levels of transcript depending on its insertion site, a phenomenon termed context sensitivity [1, 2]. Currently, it is not known what causes context sensitivity or how to engineer circuits that are isolated from the local genomic context. The study of natural genomes has given several clues to the causes of context sensitivity. Transcription of a gene is often directly controlled by its promoter and specific transcription factors (TF). This is the well-studied regulatory mechanism that involves the binding of a TF close to the promoter region which either recruits or blocks progression of RNA polymerase. This classical view of regulation fails to explain why transcription derived from both regulated and unregulated promoters have been shown to produce different levels depending on the genomic insertion site [1, 2]. Researchers speculated that for genes regulated by TFs, this may be due to the accessibility of TFs to the promoter region due to different local chromosomal architecture. Spatial organization of genes and supercoiling have been shown to influence transcription, but the regulatory mechanism controlling genes that do not have TF binding sites remains poorly defined [3].

Several factors may play a role in context sensitivity. The genome layout and orientation of transcription for a gene has been shown to impact the expression of a neighboring gene [4]. This study found that, for plasmid-encoded genes, the highest expression levels obtained were when genes are expressed towards each other. However, within bacterial chromosomes, the tandem genomic layout (co-oriented genes) is more frequently observed [1]. The transcriptional impact that different gene orientations have in a natural chromosomal context remains to be defined.

Transcription and supercoiling are tightly linked [5-7]. Liu and Wang developed the twin-supercoiled domain model of transcriptional regulation [7]. This model has since been supported by both single molecule studies and genome-wide analysis [8-10]. During transcription, RNA polymerase creates negative supercoiling upstream and positive supercoiling downstream of the sequence it transcribes [7]. The result is an accumulation of positive supercoiling downstream of a transcribed region, a phenomenon termed positive supercoiling buildup (PSB). Changes in supercoiling levels can impact transcription in many ways. For example, the tightly wound DNA may physically prevent binding of RNA polymerase and TFs. Additionally, PSB may inhibit the formation of an open complex during the initiation phase of transcription [11]. Moreover, PSB can induce pauses or stops in the initiation and elongation phases eventually leading to abortive transcription cycles. This phenomena causes transcriptional bursting for highly expressed genes, where expression has been observed to occur in a binary on/off mode [12]. Chong *et al.* showed that addition of gyrase reduces positive supercoils and significantly increases expression levels for these genes [12].

Nucleoid Associated Proteins (NAPs) are a family of DNA binding proteins that influence the architecture of smaller domains. The cellular concentration of the different NAPs has been shown to vary considerably depending upon growth phase. This causes the chromosomal architecture to dynamically change in response to different environmental conditions. These changes in chromosomal architecture have been shown to significantly impact genomic expression profiles [13-16]. These DNA binding proteins organize DNA architecture by bending, looping, bridging or wrapping of the polymer [5]. DNA loops are part of the regulatory mechanism for several genes (reviewed in [17]). For example, preventing the DNA loop formation in the lac operon has proved to reduce the repression level 70 fold [18, 19].

When the DNA double helix is subjected to high levels of torsional stress, it can self-wrap and form a plectonemic structure [20-22]. Plectoneme formation allows minimization of free energy. Regions that contain highly expressed genes are generally underwound whereas domains that carry inactive genes tend to be overwound [23]. This directly correlates the supercoiling state, either positive or negative, to gene expression. Control of PSB is critical for gene expression [24]. Topoisomerases regulate levels of supercoiling in the cell. These enzymes reduce either positive or negative supercoiling by binding DNA, cutting the phosphate backbone, either twisting or untwisting the strands before resealing the DNA [25-27]. Four different types of topoisomerases exist in *E. coli,* among them, DNA gyrase is the only enzyme that reduces positive supercoiling [28]. Gyrase is an essential enzyme that plays key functions in the cell [12, 29, 30].

At a larger scale, plectonemic supercoils organize to form what has been called a topological domain that physically isolates a DNA region [31]. When a single cut is made in a supercoiled region, it will only relax supercoiling within that protein-bound domain and not in the neighboring DNA segments. It has been shown that the binding of NAPs can prevent supercoiling from diffusing along the DNA molecule, creating a supercoiling-diffusion barrier (SDB) (a physically independent structural domain). [32]. Specifically, H-NS and Fis have been shown to contribute to the formation SDBs [33, 34]. The size of these domains have been predicted to range from 10-400 kb [5, 31, 35]. Protein-mediated DNA looping can create topological domains as well [36, 37]. It has additionally been shown that bacterial interspersed mosaic elements (BIMEs) can form SDBs [38, 39], the method of which not being understood. BIMEs are palindromic repeat sequences found between transcription units.

The inventors demonstrated that it is possible to kind of standardize the level of expression of a transgene in the genome of a cell, by isolating the transgene from the local genomic context, creating a DNA loop in which the transgene is inserted, using DNA-binding proteins that are able to dimerize or multimerize.

In particular, the inventors used a transposon mutagenesis approach to define the genomic landscape for context sensitivity in two growth conditions that are known to produce different nucleoid structures, and obtained different expression profiles from the different chromosomal architectures. They then demonstrated how encapsulating a transgene (using a reporter cassette as a proof of concept) within a protein-bound DNA loop effectively reduces context sensitivity regardless of the genomic insertion site. Using a series of synthetic constructs, it was illustrated how multiple genetic elements in different orientations within the same DNA loop impact expression levels. The inventors also showed that transcriptional inhibition of genes within the DNA loop is due to positive supercoiling buildup (PSB) that expression can be improved by incorporating a DNA sequence that is recognized by gyrase. Altogether, the inventors have defined the underlying molecular mechanisms responsible for position effects on promoter activity. The inventors defined the genomic landscape for context sensitivity and developed an approach to effectively isolate gene expression from context sensitivity. They additionally characterized how the genome layout, promoter strength, and PSB impact expression. This work provides a unifying model for a global epigenetic mechanism that is commonly used by bacteria to control a large number of genes. Evaluating a multi-Omic dataset, several examples that support the model were identified. The findings will be of particular interest for Synthetic Biologists and the engineering of standardized genetic circuits.

The invention thus relates a cell comprising, in its genome, an expression construct comprising the sequence of a gene of interest operatively linked to elements allowing its expression in the cell, wherein the expression construct is between two DNA regions that are recognized by a DNA-binding protein that is able to bind to and bridge separate DNA regions.

In view of the topology of the expression construct and the flanking sequences, such expression construct will appear in a loop, in the presence of the DNA-binding protein, that will bind to the flanking DNA regions and dimerize or multimerize so as to bridge such. Indeed, the bridging is generally obtained after self-dimerization or multimerization of the proteins that are bound to the DNA regions that they recognize. In consequence, binding of the DNA-binding protein to the DNA regions creates a DNA loop thereby isolating the expression construct from the local genomic context. The DNA loop occurs when a protein or a complex of proteins simultaneously binds to two different sites on DNA with looping out of the intervening DNA.

In the context of the invention, a "cell" can be a eukaryotic cell or a prokaryotic cell, or an archaea cell.

When the cell is a prokaryote, it may be an aerobic or anaerobic prokaryote. Among prokaryotic cells, the cell is preferably a *Escherichia coli,* a *Bacillus subtilis,* a *Lactobacillus, Pseudomonas putida, Vibrio Natriegens, Streptomyces coelicolor, Corynebacterium glutamicum, Bacillus licheniformis,* or *Bacillus amyloliquefaciens.*

A cell may also be an archaea cell. It may be of several different type of archaea that belong to the phyla *Euryarchaeota, Nanoarchaeota* or *Korarchaeota.* Within these phyla the organisms may be Phototrophs, Lithotrophs, or Organotrophs.

The cell may also be a eukaryotic cell. In particular it is a yeast cell, in particular a Saccharomyces cerevisiae cell. In another embodiment, the cell is an insect cell. In another embodiment, the cell is a plant cell, in particular a tobacco, maize, wheat, colza, soybean or Arabidopsis thaliana cell. In another embodiment, the cell is an avian cell, in particular a duck cell such as EB66 cell manufactured by Valneva (Saint Herblain, France). In yet another embodiment, the cell is a mammal cell. In particular the cell is a human cell, such as a HeLa cell, a HEK293 cell, a HT-1080 cell or a PER.C6 cell. In another embodiment, the cell is a CHO cell, a NS0 cell, a Sp2/0 cell, a BHK cell or a murine C127 cell.

In particular, any cell such as listed in Dumont et al (Crit Rev Biotechnol. 2016 Nov 1; 36(6): 1110-1122) can be used in the context of the invention. Indeed, although proof of concept was made on bacteria in the examples, the DNA-flanking regions shall be recognized by the DNA-binding protein in other organisms.

The "genome" of a cell is to be understood as the DNA elements that are transmitted to the following cell generation after mitosis or cell division. It comprises the set of chromosomes and genes of the cell, and this term includes the natural genome, as well as artificial genomes, plasmids, cosmids or artificial chromosomes (Bacterial, BAC; Yeast, YAC; or Mammalian MAC). All these artificial genomes are thus replicative by nature in order to be transmissible. Consequently, the expression vector can be included within the natural genome of the cell or within an external DNA construct (that is replicable and transmissible), which amounts the artificial genome.

In a preferred embodiment, the cell is a prokaryotic cell, in particular a bacterial cell, especially E. coli. In a preferred embodiment, the expression construct is integrated in the natural genome of the cell, especially within the bacterial genome. In another embodiment, thus, the expression construct is integrated within an artificial chromosome of the cell, in particular a Bacterial Artificial Chromosome when the cell is a prokaryote.

The expression construct comprises the sequence a gene of interest, operably linked to sequences allowing its expression in the cell.

Among genes of interest, one can cite genes coding for therapeutic proteins such as insulin, antibodies or antibody fragments, antimicrobial peptides, or for industrial enzymes such as palatase, lipozyme TL IM, lipase, lipopan, cellulose, amylase, xylose isomerase, resinase, amidase, in particular penicillin amidase, bromelain, noopazyme, asparaginase[, ficin, urokinase, β-lactamase, or subtilisin.

The elements allowing expression of a given gene sequence depend on the nature of the cell. They include promoter sequences, terminator sequences when appropriate and/or enhancer sequences.

Among promoter sequences usable in bacteria, one can cite the lac, arabinose, galactose, tetracycline, or rhamnose promoters.

Among terminator sequences that can be used in bacteria, one can cite the rrn or the trp terminator.

Among promoter sequences usable in yeast, one can cite the Gal1P, Adh, pCyc, pPGK1, GPD, ENO2 promoters.

Among terminator sequences that can be used in yeast, one can cite the STE2, ADH1, TEF1, PGK1 or CYC1 terminators.

There are multiple transcription sequences that can be used in eukaryotic cells. One can cite the transcription regulatory sequences from the Chinese Hamster EF-1alpha gene, the EEF1A (elongation factor 1 alpha)

The (at least one, as there may be more than one in the expression construct) gene of interest is located between two DNA sequences (flanking DNA sequences) that can be recognized by a protein binding to these DNA sequences and that is able, through self-dimerization or multimerization, to bridge these two DNA sequence, thereby forming a DNA loop that is isolated from the local genomic context. "Isolated from the local genomic context" means that the DNA loop becomes a DNA region independent from the epigenetic environment that are present within the region surrounding the newly formed DNA loop. Consequently, expression of the gene(s) of interest in the expression construct that is within the loop is not influenced by such regulators, and is be dependent on the regulatory sequences used with the gene(s) of interest.

One of the flanking sequences is to be recognized by and bound to a protein that is able to multimerize with another protein that has recognized and bound the other flanking sequence. It is preferred when the two flanking sequences are of the same system, i.e. are recognized by the same protein which can dimerize, although that it is foreseen that bi-functional proteins could be engineered.

Such proteins have already been described in the art.

One can cite the lambda Ci protein, as well as the sequences that can be used with this protein :

TATCACCGCCAGTGGTATTTATGTCAACACCGCCAGAGATAATTTATCACCGC AGATGGTT (SEQ ID NO: 1)

TATCACCGCAAGGGATAAATATCTAACACCGTGCGTGTTGACTATTTTACCTCT GGCGGTGATA (SEQ ID NO: 2).

In the genome of *Escherichia coli,* there are several operons that have been shown to be regulated by a small-scale DNA looping mechanism (60bp-350bp). These regulations are due to the presence of DNA sequences that are recognized by DNA-bind proteins that create loops, which would regulate action of the RNA-polymerase. Cournac and Plumbridge (Journal of Bacteriology, 2013, 195(6) 1109-1119) summarize the functioning of this regulation system. These systems are known in the art. However, they have not been proposed to be used to isolate an expression construct to express a gene of interest in a host cell. The person of skill in the art can find couples DNA-binding sequence / DNA sequences that can be used in the context of the invention, to engineer transformed cells for gene expression. It is to be noted that, in the natural context, the two DNA sequences that are recognized by the proteins/protein complex to form the loop (and that are used, in the context of the invention, as sequences flanking the gene of interest) are generally not very far apart (few dozen or hundred bp). In the context of the invention, though, these sequences flank at least one gene of interest and its regulation regions, and are thus more than 1kb, more preferably more than 2kb, more preferably more than 3kb distant.

As systems that can be or interest in the context of the invention, one can cite the fdhF (formate dehydrogenase), glnH (glutamine-binding protein), hypA (Hydrogenase Isoenzymes), prpB (Phosphoprotein phosphatase B), glnALG (regulating the nitrogen content of a cell), ara (Dunn et al 1984), lac (Oehler et al. 1994; Oehler et al. 1990), H-NS (nucleoid-associated protein, a major component of the chromosome-protein complex), LRP (leucine-responsive regulatory protein), fis (a small nucleotide-associated protein which plays a role in bacterial chromosome structure and initiation of DNA replication) operons. One can also cite the systems of transcription of gal (Haber and Adhya. 1988; Mandal et al. 1990), deo (Amouyal et al. 1989; Valentin-Hansen et al. 1986), or nag (Plumbridge and Kolb. 1991; Plumbridge and Kolb. 1993).

One can also cite the mechanisms based on the $\sigma^{54}$ bacterial enhancer-binding protein family (Ghosh et al.

2010; Morett and Segovia. 1993; Studholme and Dixon. 2003).

One can also cite bivalent dCas9 complexes as disclosed in Hao et al (2017).

This shows that multiple systems of loop-forming proteins after binding to DNA sequences are known in the art. Although a lot of these systems are of prokaryotic origin, they can be used in a prokaryotic context, as well as in a eukaryotic context, when the DNA sequences are present in the eukaryotic genome and the protein recognizing these sequences is expressed. It is also to be noted that these systems are effective naturally *in vivo* to control transcription of certain genes in their host, while they are used, in the context of the invention to generate a loop in which the gene(s) of interest is released from the local genomic context and can be expressed. In the natural state, the presence of these systems directly interacts with the RNA polymerases and thus control the level of transcription of the genes, while the invention doesn't rely on such interaction between the loops and the RNA polymerase, which will only recognize the transcription elements of the gene(s) of interest that is (are) present in the loop and thus transcribe this (these) gene(s).

It is preferred when the promoter of the gene(s) of interest is distant of at least 30 bases, more preferably of at least 50 bases of the flanking DNA regions.

In a preferred embodiment, the cell also comprises, into its genome (either the natural or an artificial genome), a gene coding for the DNA-binding protein that is able to bind to and bridge the DNA regions, operatively linked to elements allowing its expression. These elements are as known in the art and include a promoter, and optionally a terminator, enhancer regions and the like. The person skilled in the art is aware of the type of elements that are needed to allow expression of a gene in a given cell.

In a specific embodiment, the promoter that drives expression of the DNA-binding protein is an inducible promoter. Indeed, it is preferred when one is able to control expression of the protein so that it is expressed only at desirable times (for instance when the cells are grown under exponential phase, or are during stationary phase). An inducible promoter is a promoter that drives expression of the gene that it controls under specific condition. This indicates that, when these conditions are present, the expression of the gene is driven (ON state), while it is not driven if the condition is not present (OFF state). There may still be some expression driven in the OFF state, but the expression is at least 10 times lower, more preferably at least 50 times lower, most preferably at least 100 times lower in the OFF state than in the ON state. This can be determined by methods known in the art (quantification of RNA or of proteins).

Multiple inducible promoters are known in the art, and one can cite the following promoters that are effective in bacteria: GAL promoters (inducer galactose), PCUP1 (inducer $Cu^{2+}$), PADH2 (alcohol dehydrogenase 2 promoter, which displays a 100-fold reduction in expression in the presence of glucose), PPHO5 (usable in yeast, and which has a 200-fold repression in the presence of inorganic phosphate). Several synthetic inducible promoters have been developed as well such as the ones described by Liu et al (2019) or Chen et al (2018).

In plant cells, multiple inducible promoters are known in the art, with inducers such as dexamethasone, salicylic acid.

In yeast, inducible promoters are also known, and one can cite the ones disclosed by Machens et al (2017) or Xiong et al (2018).

One can note that bacterial systems have been adapted for performance in eukaryotics cells. Hence, the lac, tet. CymR/cumate, and some inducible systems involving protein-protein interactions such as (FKBP12-FRAP)/rapamycin, (PYLI-ABI1)/ABA, VVD dimerization/blue light are also usable in eukaryotic cells, and in particular mammalian cells.

As indicated above, in the natural systems, the two DNA regions are generally quite closed to each other so as to allow a small loop and control of transcription of a given gene. In the context of the present invention, though, the regions are distant enough so that at least one gene of interest is inserted or can be inserted. Consequently, the two DNA are distant of at least 1 kilobases (kb), more preferably at least 2 kb, more preferably at least 5 kb. They are generally not distant of at most 30 kb, more preferably at most 20kb. They are thus generally distant of between 1-30kb, preferably between 2-10kb.

In a particular embodiment, the two flanking DNA regions are identical. In this embodiment, the two regions can be in the same orientation. In another embodiment, the two regions are inverted.

In another embodiment, the two flanking DNA regions are different.

In a specific embodiment, only one gene of interest is present between the two DNA regions (and will thus be in the loop after recognition of these regions by the DNA-binding protein).

In another embodiment, more than one gene of interest are present between the two flanking DNA regions. These genes of interest (transgenes) can thus be expressed together when the loop is formed. The different genes of interest can all have their own transcription elements, or can be expressed as an operon, using single transcription elements to drive expression of the different genes.

In a specific embodiment, two genes of interest (transgenes) are present between the two DNA regions. In one embodiment, the two transgenes are in the same orientation (promoter 1 - gene of interest 1 followed by promoter 2 - gene of interest 2 (+ the other potential regulation elements) in this orientation). In another embodiment, the transgenes are in opposite orientation. They may be converging (promoter 1 - gene of interest 1 followed by gene of interest 2 - promoter 2) or diverging ((gene of interest 1 - promoter 1 followed by promoter 2 - gene of interest 2). It is preferred when the transgenes are in the same orientation.

The invention also pertains to a cell, comprising, in its genome, two DNA regions that are recognized by a DNA-binding protein that is able to bind to and bridge separate DNA regions. It is preferred when these regions are distant of at least 1 kilobases (kb), more preferably at least 2 kb, more preferably at least 4 kb, more preferably at least 5 kb. They are generally not distant of at most 30 kb, more preferably at most 20kb. They are thus generally distant of between 1-30kb, preferably between 2-10kb. The cell is as disclosed above. In a preferred embodiment, it is a bacterial cell, in particular an *E. coli* cell.

Such cell can serve as a template for transgenes expression. The cell may also contain two sequences, between the two DNA regions, which can be used as recognition sequences for homologous recombination. In this embodiment, the transgene(s) (or gene(s) of interest) that one wishes to express is cloned between these two recognition sequences in a specific vector, the vector is introduced in the cell and the transgene(s) is (are) introduced in the cell's genome through homologous recombination. This enables obtaining transformed cells with a specific targeting of the site of integration of the transgene and ensures that the transgene will be later isolated from the local genomic context after the looping is performed.

In a specific embodiment, the cell also contains, in its genome, a gene coding for the protein able to recognize and bridge the two DNA regions, thereby forming the loop.

The invention also related to an isolated chromosome, comprising at least one gene of interest operatively linked to elements allowing its expression, flanked by two DNA sequences that can be recognized and bridged by a DNA-binding protein, so that as to form a loop comprising the at least one gene of interest. Preferably, the isolated chromosome also contains gene cording for the protein able to recognize and bridge the two DNA regions, thereby forming the loop.

The invention also relates to a construct for transformation of a cell, comprising an expression construct comprising a promoter sequence, a gene sequence and a terminator sequence functional in the cell, wherein the expression construct is between two DNA regions that are recognized by a DNA-binding protein able to bind to and bridge these DNA regions. Such construct may be circular (such as a plasmid or a cosmid, or an artificial chromosome), or a linear construct.

The invention also relates to a method for obtaining the cell as disclosed above, comprising introducing the construct as disclosed within a host cell, so as to integrate the construct within the host cell genome.

Introduction of the construct within the cell is performed by methods known in the art, and depending on the nature of the cell, such as transformation (such as chemical transformation, electroporation, biolistics, glass beads), transduction, transfection, conjugation, lipofection.

The methods herein disclosed (introducing a transgene between two DNA regions so that the transgene is expressed after a loop has been formed through DNA-binding proteins or complexes recognizing the DNA regions) enables to perform a method for reducing transcriptional variability of a transgene introduced in a cell genome, wherein the transgene comprises a gene of interest operatively linked to elements allowing its expression, comprising introducing the transgene in the cell genome between two DNA regions that are recognized by the DNA-binding protein, and expressing the DNA-binding protein so that binding of the DNA-binding protein to the DNA regions creates a DNA loop thereby isolating the expression construct from the local genomic context. The transcriptional variability is reduced with regards to cells in which the transgene has been introduced anywhere in the genome. Indeed, as indicated above, such random transgene integration leads to great variability in the mRNA expression, whereas integration of the transgene in the isolated loop, created by the DNA-binding proteins or complexes recognizing the DNA regions, leads to essentially similar level of transcripts.

## FIGURES

Figure 1: Transposon mutagenesis to randomly insert a reporter cassette in the genome of E. coli. Tn1O mutagenesis was used to generate 209 strains. Each strain was grown in minimal media containing either glucose or glycerol as the carbon source and qPCR was used to quantify emGFP. The expression data is plotted as TPC on the y-axis to the respective genomic location on the x-axis. Green diamonds represent transcript levels for strains grown in glucose media and the red boxes are for glycerol media. Diagram representing the reporter cassette is in the upper left corner. Insert boxes have expression maps for the non-mutagenic insertions.

Figure 2: The impact of DNA looping on gene expression. The black arrows represent promoters, lollipops represent transcriptional terminators, OL and OR boxes represent lambda ci operator sites, yellow triangles represent lambda ci protein. X-axis represents the genomic loci where the constructs have been inserted. Y-axis represents emGFP TPC. A diagram of the reporter construct is illustrated. In the presence of lambda ci protein a DNA-loop is formed. Expression levels for the different strains were quantified by qPCR for growth in media containing either glycerol or glycerol and rhamnose as the carbon source.

Figure 3: Quantification of expression for synthetic cassettes at four unique genomic locations. emGFP was expressed from the strong p3 promoter. The green arrow represents the reporter gene, black arrows represent promoters, lollipops represent transcriptional terminators, OL and OR boxes represent lambda ci operator sites, yellow triangles represent lambda ci protein, bent blue box represent neutral DNA, bent brown box represent GRS. X-axis represents the 4 genomic loci where the constructs have been inserted. Y-axis represents the TPC. a) The unlooped reporter cassette. b) The reporter cassette within a DNA-loop. c) A neutral DNA sequence inserted downstream of emGFP within the DNA-loop. d) GRS downstream from emGFP in a DNA-loop. e)GRS upstream of emGFP in a DNA-loop. f) quantification.

Figure 4: Quantification for expression of two genes within a DNA loop with and without GRS. A. description of the contructs: The green and red arrows represent emGFP and mCherry genes respectively, black arrows represent promoters, lollipops represent transcriptional terminators, OL and OR boxes represent lambda cl operator sites, yellow triangles represent lambda cl protein. X-axis identifies the two genomic regions where the constructs have been inserted. Y-axis represents the number of TPC. All constructs have emGFP under the control of a strong promoter (p3) and mCherry under control of a weak promoter (p1) or a strong promoter (p10). Tandem: genes transcribed in same orientation. Convergent: genes transcribed in convergent orientation. Divergent: genes transcribed in divergent orientation. p1 bargraphs: emGFP under control of p3 and mCherry under control of p1. p10 bargraphs: emGFP under control of p3 and mCherry under control of p10. p10 (GRS) bargraphs: emGFP under control of p3, presence of GRS in the intergenic region and mCherry under control of p10. B. Quantification for expression.

Figure 5: Model of bacterial epigenetic regulation. This diagram illustrates how alternate DNA looping (through the action of different DNA binding proteins that bridge distant DNA regions) within a genomic domain would be expected to result in different transcript levels for the "B" gene.

Figure 6: Diagram of the modified transposon vector containing the reporter construct. Bent arrows represent promoters, lolipop is a terminator.

Figure 7: Quantification of terminator efficiency. a) Diagram illustrating the wild type expression and the control strain which has the mutated promoter that prevents transcription. b) Expression of *emGFP* comparing wild type expression vs expression in mutated strain in two directions in two genomic loci. Y-axis is transcripts per cell. The bent arrow represents a promoter and the red X represents a non-functional mutated promoter. lollipops are terminator structures, the boxes are lambda cl operator sequences.

Figure 8: Quantification of expression of *emGFP* and *mCherry* genes in the presence of mutants of Lambda-cl protein. a Expression of *emGFP* in locus 4,158,229. b Expression of *mCherry* in locus 4,158,229. X-axis represents the genomic locus where the construct has been inserted. Y-axis represents the TPC.

## EXAMPLES

### Example 1. Materials and methods

*Bacterial Strains, plasmids and strain construction*

Tables containing all strains, plasmids and primers used in this study are provided in following tables 1-3.

Table 1. Strains used

| Parental strain | Modified strain name | Features |
|---|---|---|
| MG1665 | BCJ952.4 | pTKRED +λcl (ΔRhaB ΔRhaA ΔRhaD) +Phleomycin |
| BCJ952.4 | α1 | pTKRED+λcl(ΔRhaB ΔRhaAΔRhaD) |
| MG1665 | BCJ2 | pTKRED+reporter cassette from pBCJ932 at locus 2,171,769 |
| MG1665 | BCJ5 | pTKRED+reporter cassette from pBCJ932 at locus 2,185,490 |
| MG1665 | BCJ7 | pTKRED+reporter cassette from pBCJ932 at locus 2,192,420 |
| MG1665 | BCJ8 | pTKRED+reporter cassette from pBCJ932 at locus 2,196,391 |
| MG1665 | BCJ12 | pTKRED+reporter cassette from pBCJ932 at locus 2,214,800 |
| MG1665 | BCJ14 | pTKRED+reporter cassette from pBCJ932 at locus 2,223,904 |

(continued)

| Parental strain | Modified strain name | Features |
|---|---|---|
| MG1665 | BCJ19 | pTKRED+reporter cassette from pBCJ932 at locus 2,244,773 |
| MG1665 | BCJ20 | pTKRED+reporter cassette from pBCJ932 at locus 2,248,654 |
| MG1665 | BCJ26 | pTKRED+reporter cassette from pBCJ932 at locus 3,959,452 |
| MG1665 | BCJ28 | pTKRED+reporter cassette from pBCJ932 at locus 3,990,959 |
| MG1665 | BCJ29 | pTKRED+reporter cassette from pBCJ932 at locus 4,035,211 |
| MG1665 | BCJ30 | pTKRED+reporter cassette from pBCJ932 at locus 4,046,827 |
| MG1665 | BCJ31 | pTKRED+reporter cassette from pBCJ932 at locus 4,158,229 |
| MG1665 | BCJ32 | pTKRED+reporter cassette from pBCJ932 at locus 4,166,287 |
| MG1665 | BCJ35 | pTKRED+reporter cassette from pBCJ932 at locus 4,207,682 |
| MG1665 | BCJ36 | pTKRED+reporter cassette from pBCJ932 at locus 4,256,593 |
| α1 | α2 | pTKRED+λcl +reporter cassette from pBCJ932 at locus 2,171,769 |
| α1 | α3 | pTKRED+λcl +reporter cassette from pBCJ932 at locus 2,185,490 |
| α1 | a5 | pTKRED+λcl +reporter cassette from pBCJ932 at locus 2,192,420 |
| α1 | α6 | pTKRED+λcl +reporter cassette from pBCJ932 at locus 2,196,391 |
| α1 | α8 | pTKRED+λcl +reporter cassette from pBCJ932 at locus 2,214,800 |
| α1 | α10 | pTKRED+λcl +reporter cassette from pBCJ932 at locus 2,223,904 |
| α1 | α12 | pTKRED+λcl +reporter cassette from pBCJ932 at locus 2,244,773 |
| α1 | α13 | pTKRED+λcl +reporter cassette from pBCJ932 at locus 2,248,654 |
| α1 | α15 | pTKRED+λcl +reporter cassette from pBCJ932 at locus 3,959,452 |
| α1 | α16 | pTKRED+λcl +reporter cassette from pBCJ932 at locus 3,990,959 |
| α1 | α17 | pTKRED+λcl +reporter cassette from pBCJ932 at locus 4,035,211 |
| α1 | α18 | pTKRED+λcl +reporter cassette from pBCJ932 at locus 4,046,827 |
| α1 | α19 | pTKRED+λcl reporter cassette from pBCJ932 at locus 4,158,229 |
| α1 | α20 | pTKRED+λcl +reporter cassette from pBCJ932 at locus 4,166,287 |
| α1 | α22 | pTKRED+λcl +reporter cassette from pBCJ932 at locus 4,207,682 |
| α1 | α23 | pTKRED+λcl +reporter cassette from pBCJ932 at locus 4,256,593 |
| α1 | α26 | pTKRED+λcl+reportercassette frompBCJ927 at2,185,490 |
| α1 | α28 | pTKRED+λcl+reportercassette frompBCJ927 at2,244,773 |
| α1 | α35 | pTKRED+λcl+reportercassette frompBCJ927 at4,046,827 |
| α1 | α36 | pTKRED+λcl+reportercassette frompBCJ927 at4,158,229 |
| α1 | α61 | pTKRED +λcl +reporter cassette from β5 at locus 2,185,490 |
| α1 | α62 | pTKRED +λcl +reporter cassette from β5 at locus 4,046,827 |
| α1 | α63 | pTKRED +λcl +reporter cassette from β5 at locus 4,158,229 |
| α1 | α64 | pTKRED +λcl +reporter cassette from β5 at locus 2,244,773 |
| α1 | α66 | pTKRED +λcl +reporter cassette from β7 at locus 2,185,490 |
| α1 | α67 | pTKRED +λcl +reporter cassette from β7 at locus 4,046,827 |
| α1 | α68 | pTKRED +λcl +reporter cassette from β7 at locus 4,158,229 |

(continued)

| Parental strain | Modified strain name | Features |
|---|---|---|
| α1 | α69 | pTKRED +λcl +reporter cassette from β7 at locus 2,244,773 |
| MG1665 | α73 | pTKRED+reporter cassette from pBCJ927 |
| | | at locus 2,185,490 |
| MG1665 | α74 | pTKRED+reporter cassette from pBCJ927 at locus 4,046,827 |
| MG1665 | α75 | pTKRED+reporter cassette from pBCJ927 at locus 4,158,229 |
| MG1665 | α76 | pTKRED+reporter cassette from pBCJ927 at locus 2,244,773 |
| MG1665 | α92 | pTKRED+reporter cassette from β5 at locus 2,185,490 |
| MG1665 | α93 | pTKRED+reporter cassette from β5 at locus 4,046,827 |
| MG1665 | α94 | pTKRED+reporter cassette from β5 at locus 4,158,229 |
| MG1665 | α95 | pTKRED+reporter cassette from β5 at locus 2,244,773 |
| α1 | α120 | pTKRED+λcl +reporter cassette from β16 at locus 2,185,490 |
| α1 | α121 | pTKRED+λcl +reporter cassette from β16 at locus 4,046,827 |
| α1 | α122 | pTKRED+λcl +reporter cassette from β16 at locus 4,158,229 |
| α1 | α123 | pTKRED+λcl +reporter cassette from β16 at locus 2,244,773 |
| α26 | α125 | pTKRED +λcl +EmGFP and p1 mCherry (Tandem) at locus 2,185,490 |
| α36 | α127 | pTKRED +λcl +EmGFP and p1 mCherry (Tandem) at locus 4,158,229 |
| α26 | α129 | pTKRED +λcl +EmGFP and p10 mCherry (Tandem) at locus 2,185,490 |
| α36 | α131 | pTKRED +λcl +EmGFP and p10 mCherry (Tandem) at locus 4,158,229 |
| α26 | α147 | pTKRED+λcl+EmGFP and p1 mCherry (Convergent) at locus 2,185,490 |
| α36 | α148 | pTKRED+λcl+EmGFP and p1 mCherry (Convergent) at locus 4,158,229 |
| α26 | α149 | pTKRED+λcl+EmGFP and p10 mCherry (Convergent) at locus 2,185,490 |
| α36 | α150 | pTKRED+λcl+EmGFP and p10 mCherry (Convergent) at locus 4,158,229 |
| α1 | α153 | pTKRED+λcl +reporter cassette from β22 at locus 2,185,490 |
| α1 | α154 | pTKRED+λcl +reporter cassette from β16 at locus 4,158,229 |
| α125 | α165 | pTKRED+λcl+EmGFP and p1 mCherry (Divergent) at locus 2,185,490 |
| α127 | α166 | pTKRED+λcl+EmGFP and p1 mCherry (Divergent) at locus 4,158,229 |
| α129 | α167 | pTKRED+λcl+EmGFP and p10mCherry (Divergent) at locus 2,185,490 |
| α131 | α168 | pTKRED+λcl+EmGFP and p10mCherry (Divergent) at locus 4,158,229 |
| α26 | α169 | pTKRED+λcl+EmGFP and mCherry (Tandem mutated) at locus 2,185,490 |
| α36 | α170 | pTKRED+λcl+EmGFP and mCherry (Tandem mutated) at locus 4,158,229 |
| α171 | α179 | pTKRED +λcl +EmGFP, GRS and p10 mCherry (Tandem) at locus 2,185,490 |
| α172 | α180 | pTKRED +λcl +EmGFP, GRS and p10 mCherry (Tandem) at locus 4,158,229 |
| α173 | α181 | pTKRED+λcl+EmGFP,GRS and p10 mCherry (Convergent) at locus 2,185,490 |
| α174 | α182 | pTKRED+λcl+EmGFP,GRS and p10 mCherry (Convergent) at locus 4,158,229 |
| α175 | α183 | pTKRED+λcl+EmGFP,GRS and p10 mCherry (Divergent) at locus 2,185,490 |
| α176 | α184 | pTKRED+λcl+EmGFP,GRS and p10 mCherry (Divergent) at locus 4,158,229 |

(continued)

| Parental strain | Modified strain name | Features |
|---|---|---|
| α180 | α240 | pTKRED +λcl (Y210H) +EmGFP, GRS and p10 mCherry (Tandem) at locus 4,158,230 |
| α180 | α241 | pTKRED+λcl(S228R) +EmGFP,GRS and p10 mCherry (Tandem) at locus 4,158,231 |
| α180 | α242 | pTKRED+λcl(P158T) +EmGFP,GRS and p10 mCherry (Tandem) at locus 4,158,232 |

**Table 2. plasmids used**

| Name | Content |
|---|---|
| pBCJ932 | Lambda-cl boxes, p1-EmGFP |
| pBCJ927 | Lambda-cl boxes, p3-EmGFP |
| pBCJ827.4 | Phleomycin Resistant, Lambda-cl boxes, p3-EmGFP |
| pBCJ879.2 | Phleomycin Resistant, p3-EmGFP |
| pBCJ937.1 | Lambda-cl integration vector |
| pJet2.1 | commercial cloning plasmid |
| β5 | Lambda-cl boxes, p3-EmGFP,GRS (downstream EmGFP) |
| β7 | Lambda-cl boxes, p3-EmGFP,Neutral DNA (downstream EmGFP) |
| β9 | Lambda-cl boxes, GRS (upstream EmGFP), p3-EmGFP |
| β16 | Lambda-cl boxes, p3-EmGFP, GRS (outside loop) |
| β22 | pBCJ927 plasmid with mutated P3 promoter |
| β23 | Mutated promoter+mCherry+Lambda-cl binding box+ |

**Table 3. Primers**

| SEQ ID | | Sequence (5'-3') | Binding site |
|---|---|---|---|
| 3 | 147F | TGAATTCATCGTCATTTACCCATATTCAATTGTGGCTAGTGTAAACGAAGTACGGCCCCAAGGTCCAAAC | Binds pBCJ927/pBCJ932 targeting location 2 171 769 on genome |
| 4 | 147R | GTGATGCTGGCCCGGTATTGTGCAAAACAGATCATTCACCAATGGTCCCCTTGGCTTCAGGGATGAGGCG | Binds pBCJ927/pBCJ932 targeting location 2 171 769 on genome |
| 5 | 150F | GATAAATCGCAGAGGAGGATGGTAATGTCCAGCGCACGCGTTGTAAACGATACGGCCCCAAGGTCCAAAC | Binds pBCJ927/pBCJ932 targeting location 2 185 490 on genome |
| 6 | 150R | TTCATATATCAAATAATTTATTAACGCGATTGTAAAACTGCCGTTTTTCCTTGGCTTCAGGGATGAGGCG | Binds pBCJ927/pBCJ932 targeting location 2 185 490 on genome |

(continued)

| SEQ ID | | Sequence (5'-3') | Binding site |
|---|---|---|---|
| 7 | 152F | AATATTTAAGAGTATTAACTATTTATCGCATCTATCAATTAATGTAGATTTACGGCCCCAAGGTCCAAAC | Binds pBCJ927/pBCJ932 targeting location 2 192 420 on genome |
| 8 | 152R | ATATAAATGATTTCGGCTTTTTTATTGATATCAACAATACCATTTACATATTGGCTTCAGGGATGAGGCG | Binds pBCJ927/pBCJ932 targeting location 2 192 420 on genome |
| 9 | 153F | CATCGACAGCGCCTTTTCTTTATAAATTCCTAAAGTTGTTTTCTTGCGATTACGGCCCCAAGGTCCAAAC | Binds pBCJ927/pBCJ932 targeting location 2 196 391 on genome |
| 10 | 153R | CAGTTGAATGCAGATGCTACCAGTATTTATGCGGGTTAGAGAGAGACAAATTGGCTTCAGGGATGAGGCG | Binds pBCJ927/pBCJ932 targeting location 2 196 391 on genome |
| 11 | 157F | AAATCGCCTGGCAAAAATAAAATCACCCTATAGATGCACAAAAAAACGGGCTACGGCCCCAAGGTCCAAAC | Binds pBCJ927/pBCJ932 targeting location 2 214 800 on genome |
| 12 | 157R | GCGAGGTCCCGGTTTAACTTTAGACGCAGTTTTGCGAACCAGGTAGTTTTTTGGCTTCAGGGATGAGGCG | Binds pBCJ927/pBCJ932 targeting location 2 214 800 on genome |
| 13 | 159F | GGATCGGTAAAACCAGTAAACGGAAAAACTGGCAGGAAGTGGAGTAAAAATACGGCCCCAAGGTCCAAAC | Binds pBCJ927/pBCJ932 targeting location 2 223 904 on genome |
| 14 | 159R | GGCACAGAACGATTAAGTGAATTCGGATGGCGATACTCTGCCATCCGTAATTGGCTTCAGGGATGAGGCG | Binds pBCJ927/pBCJ932 targeting location 2 223 904 on genome |
| 15 | 164F | CATGCGCAGTATTTACTGAAGTGAAAGTCCGCCCGGTTCGCCGGGCATCTTACGGCCCCAAGGTCCAAAC | Binds pBCJ927/pBCJ932 targeting location 2 244 773 on genome |
| 16 | 164R | GAAGACGGACGTCGTTACTTTATGGCAGTGGATTATCGCTTCTGATGAGATTGGCTTCAGGGATGAGGCG | Binds pBCJ927/pBCJ932 targeting location 2 244 773 on genome |
| 17 | 165F | ACACGGTTATAAGACACCTTCATGATCGCCCAGGGATTATAAGTAAAGCATACGGCCCCAAGGTCCAAAC | Binds pBCJ927/pBCJ932 targeting location 2 248 654 on genome |
| 18 | 165R | ATTTCGTGACGCAGCGCCTTCAGCATGCATTCGCCAGAAAAGAGATTGGCTTGGCTTCAGGGATGAGGCG | Binds pBCJ927/pBCJ932 targeting location 2 248 654 on genome |

(continued)

| SEQ ID | | Sequence (5'-3') | Binding site |
|---|---|---|---|
| 19 | 245F | GAAACTGCGCGGCTATATGACAGATATGAAACGTATTGCTGTTGCGGGTTAAGTGCGCGCTACGGCCCCAAGGTCCAAAC | Binds pBCJ927/pBCJ932 targeting location 3 959 452 on genome |
| 20 | 245R | AGGAGAAGGCCTTGAGTGTTTTCTCCCTCTCCCTGTGGGAGAGGGTCGGGGTGAGGGCATCATTGGCTTCAGGGATGAGGCG | Binds pBCJ927/pBCJ932 targeting location 3 959 452 on genome |
| 21 | 247F | AATAAGGAATTTACAGAGAATAAACGGTGCTACACTTGTATGTAGCGCATCTTACGGCCCCAAGGTCCAAAC | Binds pBCJ927/pBCJ932 targeting location 3 990 959 on genome |
| 22 | 247R | ATGGTCTAAAACGTGATCAATTTAACACCTTGCTGATTGACCGTAAAGAATTGGCTTCAGGGATGAGGCG | Binds pBCJ927/pBCJ932 targeting location 3 990 959 on genome |
| 23 | 248F | AAGCATAAAGAATAAAAAATGCGCGGTCAGAAAATTATTTTAAATTTCCTCTTGTCAGGCTACGGCCCCAAGGTCCAAAC | Binds pBCJ927/pBCJ932 targeting location 4 035 211 on genome |
| 24 | 248R | CCGGCGGCGTGTTTGCCGTTGTTCCGTGTCAGTGGTGGCGCATTATAGGGAGTTATTCCGTTGGCTTCAGGGATGAGGCG | Binds pBCJ927/pBCJ932 targeting location 4 035 211 on genome |
| 25 | 249F | ACAAACATCATGCTGTAAAAAGCATGATAATAAATTAAAAGCGATGTAAATAATTTATGCTACGGCCCCAAGGTCCAAAC | Binds pBCJ927/pBCJ932 targeting location 4 046 827 on genome |
| 26 | 249R | TCGTGAAAATCTTTTGTAGATCTTCTGGATCGCTCGCAAATCGTCATGTGGATAACTTTGTTTGGCTTCAGGGATGAGGCG | Binds pBCJ927/pBCJ932 targeting location 4 046 827 on genome |
| 27 | 250F | TCACCGCAGCAGGTGGCGCAGGCGATTGCTTTTGCGCAGGCTCGGTTAGGGTAAGAACATTACGGCCCCAAGGTCCAAAC | Binds pBCJ927/pBCJ932 targeting location 4 158 229 on genome |
| 28 | 250R | CCGCTTTTTTTTGCCATAAAAAAGCCCGGCGATAAGCCAGGCTCAAATTTATACATATAATTGGCTTCAGGGATGAGGCG | Binds pBCJ927/pBCJ932 targeting location 4 158 229 on genome |
| 29 | 251F | TATTGCCCGTTTTACAGCGTTACGGCTTCGAAACGCTCGAAAAACTGGCAGTTTTAGGCTGATTTACGGCCCCAAGGTCCAAAC | Binds pBCJ927/pBCJ932 targeting location 4 166 287 on genome |

(continued)

| SEQ ID | | Sequence (5'-3') | Binding site |
|---|---|---|---|
| 30 | 251R | AAATAATTTTCTGACCGCGCAACATTCAACCAAATCAGCCTAAAACTGCCAGTTTTTCGAGCTTGGCTTCAGGGATGAGGCG | Binds pBCJ927/pBCJ932 targeting location 4 166 287 on genome |
| 31 | 254F | TTTTCGCCCGCATTGTAACGAAAACGTTTGCGCAACGCTCGCGAATTTTTCTCTTTCAATTACGGCCCCAAGGTCCAAAC | Binds pBCJ927/pBCJ932 targeting location 4 207 682 on genome |
| 32 | 254R | AATCATCAATGTAATTTCTGTATTTTGCCCACGGTAACCACAGTCAAAATTGTGATCACCTTGGCTTCAGGGATGAGGCG | Binds pBCJ927/pBCJ932 targeting location 4 207 682 on genome |
| 33 | 255F | TCTGTGGTATCCGCTCATGTTTCGCGCGGCGCTACGCAAACCCGAATCATCGGATTTAACTACGGCCCCAAGGTCCAAAC | Binds pBCJ927/pBCJ932 targeting location 4 256 593 on genome |
| 34 | 255R | GTGGTATTATTGGCCATTGAAAGAACCTTTTTACATTATGAGCGTCAATATCAGTGTACCTTGGCTTCAGGGATGAGGCG | Binds pBCJ927/pBCJ932 targeting location 4 256 593 on genome |
| 35 | 147s1 | TACTCCCGCCAGATCCTGAA | Genomic control of insertion of Construction pBCJ927/pBCJ932 |
| 36 | 147s2 | AGCCATTAATCGCTCACCGT | |
| 37 | 150s1 | CGAGTCGTTTGGTTGCGATG | Genomic control of insertion of Construction pBCJ927/pBCJ932 |
| 38 | 150s2 | AACACATCGTTCACCAGGGG | |
| 39 | 152s1 | GCGGCAGCAATAATTGAACG | Genomic control of insertion of Construction pBCJ927/pBCJ932 |
| 40 | 152s2 | GAAGTGGTTGTTGCAGGTAGC | |
| 41 | 153s1 | ATAATCCCCCTTCAAGGCGC | Genomic control of insertion of Construction pBCJ927/pBCJ932 |
| 42 | 153s2 | GCAACTCGTTTCCCTGTTGC | |
| 43 | 157s1 | TACCCGCAAACCACACTTCC | Genomic control of insertion of Construction pBCJ927/pBCJ932 |
| 44 | 157s2 | CAAAGCAACGCCACTTCACC | |
| 45 | 159s1 | CGACCAACAGCATGAATCCG | Genomic control of insertion of Construction pBCJ927/pBCJ932 |
| 46 | 159s2 | ACAAAAAGCCGCCCAAATGG | |
| 47 | 164s1 | GATGTATGCCAGTAACGCGC | Genomic control of insertion of Construction pBCJ927/pBCJ932 |
| 48 | 164s2 | ACTATACCGGGCAGAAACGC | |
| 49 | 165s1 | AAATGAGGCGCGATTGTAGC | Genomic control of insertion of Construction pBCJ927/pBCJ932 |
| 50 | 165s2 | GGCGCTTTCTGGACTATTGC | |
| 51 | 245s1 | CTTGTGTGCCGTTGCTGAAA | Genomic control of insertion of Construction pBCJ927/pBCJ932 |
| 52 | 245s2 | TGGTTCCGGCGTTCGATAAA | |
| 53 | 247s1 | TGGATCCTGACAGGCGTTTC | Genomic control of insertion of Construction pBCJ927/pBCJ932 |
| 54 | 247s2 | TTGGAATGCAGGCCCCATAG | |

(continued)

| SEQ ID | | Sequence (5'-3') | Binding site |
|---|---|---|---|
| 55 | 248s1 | GTCAGGCGGTGAAACGGATA | Genomic control of insertion of Construction pBCJ927/pBCJ932 |
| 56 | 248s2 | TTTGCTTTCTCTGCCGGAGT | |
| 57 | 249s1 | GCAAGGCGCAGATTTTAGCA | Genomic control of insertion of Construction pBCJ927/pBCJ932 |
| 58 | 249s2 | GATCTTCTGGATCGCTCGCA | |
| 59 | 250s1 | CGATTCTGTCGCTGCAATCG | Genomic control of insertion of Construction pBCJ927/pBCJ932 |
| 60 | 250s2 | ACTGCCCGTTTCGAGAGTTT | |
| 61 | 251s1 | TTACAAGTGCTGCCAGAGGG | Genomic control of insertion of Construction pBCJ927/pBCJ932 |
| 62 | 251s2 | CCGGCCTGACAAGAGGAAAT | |
| 63 | 254s1 | TTCGACGATACCGGCTTTGT | Genomic control of insertion of Construction pBCJ927/pBCJ932 |
| 64 | 254s2 | TGACTGACTGCTGCATTCCC | |
| 65 | 255s1 | AGAGGTATCCAGCCCCAGTT | Genomic control of insertion of Construction pBCJ927/pBCJ932 |
| 66 | 255s2 | CTTCGTTGATCCATGCAGCG | |
| 67 | 90a | GAGAGATGGGTAAGCACAAC | Lambda-cl genome insertion control |
| 68 | 263F | TCAGGTGGCTCATCACGCTA | |
| 69 | 92c | TCGTGACCACCTTGACCTAC | EmGFP qPCR probes |
| 70 | 92d | GTCGTCCTTGAAGAAGATGG | |
| 71 | A78F | GGATACACATCTTGTCATATGTAT CACCGCCAGTGGTATTTATGTCAA CACCGCCAGAGATAATTTATCACC GCAGATGGTTTACGGCCCCAAGG TCCAAAC | Overhangs containing lambda-Cioperator boxes |
| 72 | A78R | GGATACACATCTTGTCATATGTAT CACCGCCAGAGGTAAAATAGTCA ACACGCACGGTGTTAGATATTTAT CCCTTGCGGTGATATTGGCTTCA GGGATGAGGCG | |
| 73 | A115F | TCACCCGGGTCGTATAATGTGTG GATACTAGTGAAAGAGGAGAAAT ACT | Overhangs containing strong promoter p3 for pBCJ927 |
| 74 | A115R | CGACCCGGGTGAATTAATAAGCA AATAAATTGTCGCTATTGAGTGAG CTAACTCACA | |
| 75 | A143R | ATTCAGGCGCTTTTTAGACTGGTC GTAATGAAATTCAGCAGGATCACC ATATGAGCACAAAAAAGAAACCAT TAAC | Insertion of Lambda-CI in Rha operon |
| 76 | A144R | GCTCCTTTGTCTGTCGTGTAGAGT GAATCTGCGCCACATTTGGCTTCA GGGATGAGGCG | |
| 77 | N1F | GCTTGATATCGAATTCCGAAG | Insertion of GRS downstream emGFP |
| 78 | N1R | CTTGTACAGCTCGTCCATGC | |

(continued)

| SEQ ID | | Sequence (5'-3') | Binding site |
|---|---|---|---|
| 79 | N2F | GGCCGATTCATTAATGCAGCTGG CACGAC | Insertion of GRS upstream emGFP |
| 80 | N2R | AACGCGCGGGGAGAGGCGGTTT GCGTATT | |
| 81 | N3F | TAGCTTGGCTGCAGGTCGTCGAA ATTC | Insertion of GRS downstream the loop |
| 82 | N3R | GCTTGGCTGGACGTAAACTC | |
| 83 | N6F | CATGGTCATAGCTGTTTCCAGCG CCGGAAGTTGTGTAACAGTCATG CCCGGCGTTCTGGTCCATTC | Insertion neutral DNA downstream the loop |
| 84 | N6R | ACTGGCCGTCGTTTTACACACCA GGTTTACCTATCGTTCTGATTGCA TATCCGGTAACTGCGG | |
| 85 | N16F | TACGGCCCCAAGGTCCAAACG | Binds on Landing pad 1 |
| 86 | N16R | TTGGCTTCAGGGATGAGGCG | Binds on Landing pad 2 |
| 87 | N53F | GCATCCGGGCGTGCGCTCTATAC TAGTGAAAGAGGAGAAATACTAG ATGGTAAGCAAGGGCGAG | For mutation of p3 promoter |
| 88 | N53R | TAGAGCGCACGCCCGGATGCGAT GAATGTGCTATGGACCATAAGGT CGCTACGGGACCTGCTAACAACG TTAGAGCCTGTAACTGCGCCTG | |
| 89 | N64F | TGTAACTCACTCAATAGCGACGC GAGTTGTAACGCTCCAGCTGCTA GTAGATAGACATAGCTCTAGGCG TACTAGTGAAAGAGGAGAAATACT AGATGGTGAGCAAGGGCGAGG | mCherry from plasmid pMC48 with mutated promoter |
| 90 | N19R | GCATCTTCGGCATTTTTGCCCCAT GCAAACGGGCCGTGGGAATGGA CCAGAACGCCGGGCAATAAAAAA GCCCCCGGAATGATCTTCCGGGG GCTTACTTGTACAGCTCGTCCATG CC | |
| 91 | N64R | CTCGCGTCGCTATTGAGTGAGTT ACA | pBCJ927 for integration of mutated promoter mCherry |
| 92 | N22R | CGCCTCATCCCTGAAGCCAA | |
| 93 | N80F | GAACGGCCACGAGTTCGAGA | mCherry qPCR probes |
| 94 | N80R | CTTGGAGCCGTACATGAACTGAG G | |
| 95 | N84F | GTGGGGAAAGTTATCGCTCGCCA GTGGCCTGAAGAGACGTTTGG | Insertion of S228R mutation in Lambda-cl |

(continued)

| SEQ ID | | Sequence (5'-3') | Binding site |
|---|---|---|---|
| 96 | N86F | CACTAAACCCACAGCATCCAATGA TCCCATGCAATGAGAGTTGTTC | Insertion of Y210H mutation in Lambda-cl |
| 97 | N92F | TGAGGTTGAAGGTAATTCCATGAC CGCACCAACAGGCTCCAAGATCC CGCCGAAAGGCGGGATTTTCTTA GGGATAACAGGGTAATAGTTGAC AATTAATCATCGGCATAG | Insertion of P158T mutation in Lambda-cl |
| 98 | pUCF | CCCAGTCACGACGTTGTAAAACG | Amplification of GRS |
| 99 | pUCR | AGCGGATAACAATTTCACACAGG | |

*Plasmid construction*

To define the genomic landscape for context sensitivity a library of strains as constructed, that had a single reporter cassette randomly inserted into different genomic locations. Vectors used for tn10 mutagenesis were modified as illustrated in Figure 6. Briefly, pNKBOR plasmid was modified so that the RK6 gamma ori would not be incorporated into the genome. All cloning steps for constructs containing the RK6 gamma ori were done using DH5α-pir. A second IS101 site was inserted between the transposase and the kanamycin resistance gene resulting in pNK2. A synthetic DNA fragment (from IDT-DNA) was then ligated into the NotI site of pNK2 to make transposon mutagenesis vector pBCJ827.4 (Figure 6).

To add the DNA binding sites for lambda CI to each side of the reporter cassette a template vector was constructed for generating PCR products that would be used for lambda red integration. Initially, plasmid pTKIP was modified by swapping the kanamycin cassette with a pheomycin resistance cassette. pTKIP was digested with BamHI and the phleomycin resistance gene (obtained as synthetic fragment from IDT-DNA) was ligated into the corresponding site resulting in pBCJ879.2. Lambda CI boxes were added to the reporter cassette by PCR of pBCJ827.4 as oligo overhangs on primers A78F & A78R. pBCJ879.2 was then KpnI and cloned with the emGFP reporter cassette PCR fragment using NEB HiFi (using manufacturer's protocol) resulting in pBCJ932. To express emGFP from a strong promoter (P3) inverse PCR (primers A115F & A115R) of pBCJ932 were used resulting in pBCJ927.

To construct a strain of *E. coli* that had the lambda CI protein expressed from the inducible rhamnose promoter lambda red was used. This required the construction of a plasmid that could be used as a template for a PCR product. The lambda CI gene was purchased as a synthetic DNA fragment from IDT-DNA and inserted into the KpnI site of pBCJ879.2 resulting in plasmid pBCJ937.1.

To construct plasmid β5 carrying the GRS downstream emGFP, plasmid pBCJ927 was amplified by inverse PCR using primers N1F & N1R. A synthetic DNA fragment carrying the GRS flanked by 20bp homologous pads to insertion site (from IDT-DNA) was inserted into the plasmid pBCJ927 by ligation (T4 DNA Ligase, NEB) resulting in plasmid β5. Same approach was used for plasmids β9 and β16, using primers N2F & N2R and N3F & N3R respectively for inverse PCRs on plasmid pBCJ927.

To construct plasmid β7 carrying the neutral DNA downstream emGFP, plasmid pBCJ927 was amplified by inverse PCR using primers N1F and N1R.

To construct plasmid β23, primers N64F & N19R were used to amplify mCherry gene from plasmid pMC48. Primer N64F binds at the beginning of mCherry and has an overhang that contains a random 44bp sequence (as a replacement for p1 promoter) and a 20bp sequence homologous to insertion site in backbone plasmid. Primer N19R binds at the end of mCherry gene and carries a terminator and a 20 bp homologous sequence for insertion in backbone plasmid. Primers N64R & N22R were used to amplify the backbone plasmid from plasmid pBCJ927. The two PCR fragment were then ligated to get plasmid β23.

To construct plasmid β22 carrying emGFP with mutated p3 promoter, plasmid pBCJ927 was amplified by inverse PCR using primers N53F & N53R. These primers carry overhangs containing a replacement sequence for p3 and 20bp homologous sequences to each other. The PCR fragment was ligated to itself resulting in plasmid β22.

The plasmid pMC48 was constructed by inserting a DNA fragment purchased from IDT-DNA encoding a gentamycin resistance gene into pJet2.1.

*Strain construction*

To engineer a strain that has the lambda CI gene expressed from the inducible rhamnose promoter lambda red was used. PCR of pBCJ937.1 with primers (A143R & A144R) generated a DNA fragment that directly replaces the *rhaB* gene with lambda CI and a pheomycin resistance marker. Integration of this fragment into the *E. coli* resulted in BCJ952.4. The phleomycin resistance marker was subsequently removed by lambda red integration of a cassette carrying a neomycin resistance gene which was then excised from the loci resulting in α1.

Lambda red integration was used to construct a series of strains that have emGFP gene expressed by the weak p1 promoter flanked by lambda cl binding sites. PCR fragments were generated using pBCJ932 as template and a series of primers (Table 3) that targeted the cassette to different genomic locations. Lambda red genomic integrations were performed as described previously [65, 66]. To construct strains (2 to 36) the PCR fragments were integrated into *E. coli* MG1655, and for strains (α2 to α23), the PCR fragments were integrated into α1.

Lambda red was used to construct strains that have emGFP expressed by the strong P3 promoter flanked by lambda CI binding sites. Plasmid pBCJ927 was used as PCR template to construct strains (α28 to α36). To engineer strains α61 to α64 lambda red recombineering was used. PCR reactions using primers 150F-R, 164F-R, 249F-R & 250F-R (Table 3) were performed on plasmid β5. These amplifications were then inserted in the target genomic loci using lambda red recombination.

To build strains α66 to α69 PCR reactions using primers 150F-R, 164F-R, 249F-R & 250F-R (Table 3) were performed on plasmid β7. These amplifications were then inserted in the target genomic loci using lambda red recombineering .

To build strains α120 to α123 PCR reactions using primers 150F-R, 164F-R, 249F-R & 250F-R (Table 3) were performed on plasmid β16. These amplifications were then inserted in the target genomic loci using lambda red recombineering .

To build strains α125 to α150 and α165 to α168, primers N19F, N21F, N23R, N39R, N43F, N46F, N65R (Table 3) were used to amplify mCherry from plasmid 48. When needed, these primers have an overhang containing a replacement sequence for the promoter part. These amplifications were then inserted in the target genomic loci using lambda red recombineering in strains α26 or α36.

To build strains α153 and α154 PCR reactions using primers 150F-R & 250F-R (Table 3) were performed on plasmid β22. These amplifications were then inserted in the target genomic loci using lambda red recombineering.

To build strains α169 and α170, PCR reactions using primers N22R & N64R (Table 3) were performed on plasmid β23. These amplifications were then inserted in the target genomic loci using lambda red recombineering.

To build strains α179 to α184, PCR reactions using primers listed in Table 3 were performed on plasmid β5, amplifying only the GRS. The PCR product was then inserted in the target genomic loci in strains α129, α131, α149, α150, α167, or α168 using lambda red recombineering.

To build strains α240 to α242 expressing mutants of lambda-cl protein, a PCR product containing chloramphenicol resistance gene amplified from pMC48 using primers N88F-R (Table 3) was inserted in *E. coli* genome, truncating lambda-cl gene. A second PCR product (obtained from primer N84F ; N86F or N92F), containing the P158T, Y210H or S228R mutation was introduced in replacement of chloramphenicol resistance gene using lambda red recombineering. Colonies sensitive to chloramphenicol were selected. The sequence of the lambda cl mutants were verified by PCR and sequencing.

The primers and genetic constructs were designed using MacVector software and EcoCyc [67].

*Cell growth conditions*

Luria Broth (LB) was used for the routine growth of *E. coli* strains (31). M9 media with glycerol was used for the growth of strains for RNA extraction and qPCR measurements. M9 media is 6 g/L Na2HPO4 x 2H2O, 3 g/L KH2PO4, 0.5 g/L NaCl, 0.002% Casamino acids, 2 mM MgSO4, 100 μM CaCl2 and 0.8% glycerol as the carbon source. Antibiotics were added to the culture as needed at the following concentrations: spectinomycin (60μg/mL), kanamycin (25μg/mL genomic integrations, 50μg/mL for plasmids), phleomycin (10μg/mL), gentamicin (10μg/mL), and ampicillin (100ug/ml). Bacterial cultures were grown at 30°C or 37°C with 200 rpm agitation.

*Molecular biology methods*

For routine PCR amplification, Q5 and OneTaq DNA Polymerase was used per manufacturer's supplied protocol (NEB®). PCR products were cleaned using Monarch DNA CleanUp Kit from NEB®, plasmids were purified using Monarch Plasmid Miniprep Kit, following manufacturer's supplied protocols. PureLinkTM Genomic DNA Extraction Kit was used to extract genomic DNA (ThermoFisher®). Simply Seamless DNA Assembly Kit was used to assemble synthetic DNA fragments and clone, following manufacturer's supplied protocols.

Electrocompetent cells were prepared as described previously [68]. Briefly, bacterial strains were grown overnight in LB containing the appropriate antibiotics. This seed culture was then diluted 1:400 to inoculate 200mL of LB containing antibiotics and IPTG if necessary, and grown at 30°C until reaching OD600 ~ 0.5. Cell pellets were harvested by centrifugation at 3,900 xg at 4°C for 6 minutes, and washed 2 times with an equal volume of ice cold 10% glycerol and then resuspended in 2mL 10% glycerol.

Electroporations were performed on an Eppendorf 2510 using manufacturer-supplied protocols. Prior to electroporations performed for lambda red, all PCR fragments were digested with DpnI for 1-hour to remove template plasmid. 200ng of purified DNA fragment containing the linear construct to be integrated was mixed with 50μL electrocompetent cells in 0,1 cm electroporation cuvettes. Cells were electroporated at 1,8 kV and immediately resuspended in 1mL LB, incubated shaking 200 rpm, 3h at 30°C, and 100μL was plated onto LB containing the antibiotics of interest and incubated at 30°C overnight. Genomic DNA was extracted from putative colonies, PCR was used to amplify the genomic region of the integration and all strains were confirmed by sequencing.

Routine agarose gel electrophoresis was conducted in RunOne Electrophoresis System (Embitec) using; Thermo Scientific Loading Dye, a 1% or 2% agarose gel, 1X TBE (Tris, Boric Acid, EDTA) buffer and run for 20 minutes at 100V. Gels were stained with Ethidium Bromide (Sigma) for RNA and Midori Green (Nippon Genetics) for DNA, before being visualized under UV light or using a G-box iChemi (Syngene).

*Western Blot Analysis*

Proteins were extracted from 5mL cultures at $OD_{600}$ = 0.5 in M9 glycerol media containing different concentration of Rhamnose using BugBuster Reagent Protein Extraction Kit (Novagen). Protein concentration of cell lysates was quantified using the Bradford assay (Sigma). 40ug of total protein extract was loaded per lane in a pre-made gradient polyacrylamide sodium dodecyl sulfate gel (Bio-Rad) with PrecisionPlus protein marker (Bio-Rad) and run for 1h at 80V in a Mini-PROTEAN Tetra Cell (Bio-Rad). The separated proteins were transferred on a 0.45μm Immobilon PVDF Membrane filter (Millipore). The filter was first incubated in Blocking Buffer (NaCL, Marvel milk, TBS) for 1 hour with 2μL/mL primary antibody (anti-Flag, produced in rabbit, Sigma), washed with TBST, and incubated for 1 hour with 0.1μL/mL secondary antibody (HRP conjugated anti-rabbit IgG, Sigma). ECL Western Blotting Detection Reagents kit (GE healthcare) was used for detection per manufacturer's supplied protocol. Image processing was performed on G-box iChemi.

*RNA Isolation*

Cells were inoculated to 300μL M9 Glycerol media and grown overnight at 37°C. The following day, 10mL of M9 Glycerol was inoculated with 200μL of overnight culture. 10mM Rhamnose was added to the cultures as needing if expression of lambda cl is required. The samples were grown until they reach OD600 ~ 0.55 and harvested by centrifugation, 10 minutes at 3,900 xg at 4°C. The pellets were snap frozen in dry ice/ethanol bath and stored at -80°C until RNA was extracted. To extract total RNA, cell pellets were transferred to ice and resuspended in 1mL of Ribozol RNA Extraction Reagent (VWR). RNA extraction was performed per manufacturer's supplied protocol. The final RNA pellets were resuspended in approximately 225μL of water, depending upon pellet size. RNA was treated with DNase I (NEB) per company supplied protocol. RNA was then precipitated by adding of 20μL sodium acetate and 500μL of isopropanol. RNA was then pelleted at 21,130 xg for 30 minutes, washed in 500μL of 75% Ethanol, and resuspended in 80μL of water. The integrity, quality and quantity of purified RNA was determined by agarose gel electrophoresis and nanodrop measurements.

*Reverse-Transcription and Quantitative PCR (RT-qPCR)*

Five hundred nanograms of RNA was used to perform Reverse Transcription using Protoscript II RT Kit per manufacturer's supplied protocol (New England BioLabs®). While conducting this large qRT-PCR study we discovered that there is a significant batch to batch variation in Reverse Transcriptase. To prevent this from impacting the datasets, all of the qRT-PCR reagents used during the entire study came from a single production batch. cDNA samples were purified using GeneJET PCR Purification Kit (Thermofisher®), and eluted in 50μL final volume. cDNA samples were diluted 10 times, and qPCR was performed using SYBR Premix Ex Taq Kit (Takara) per manufacturer's supplied protocol. Primers used to quantify expression for the different genes are in Table 3. Quantitative PCR was performed on Realplex$^2$ Mastercycler from Eppendorf® using manufacturer's supplied protocol and the following optimized parameters: 40 cycles with denaturation 5 seconds at 95°C, primer annealing for 30 seconds at 60°C, and extension at 72°C for 20 seconds. An external standard (a dilution series for the corresponding PCR product) was added to each qPCR plate. All samples were measured in duplicate on the plate. All measurements were the average of a minimum of three independent cultures. The standard error was less than 30% for all averaged values.

*Statistics and data analysis*

Absolute quantification of gene targets were performed using absolute quantitation via a standard DNA curve.

Data analysis was done with Microsoft Excel. To obtain the number of transcript per cell, the following formula was used:

$$\text{Number of copies} = (\text{X nanograms} * \text{Avogadro's number}) / (\text{molecular weight} * 1\text{x}10^9)$$

X corresponds to the amount of amplicon got from qPCR, Avogrado's number ($6.0221\text{x}10^{23}$) corresponds to the number of molecules per mole, molecular weight of EmGFP is 233703.9, molecular weight of mCherry is 231269.38, multiplied by $1\text{x}10^9$ to get the number of molecules per nanogram of total RNA. This number is then divided by 10,000 to obtain the number of molecules per cell.

**Example 2. Context sensitivity of transcription all along the E. *coli* chromosome**

To obtain a global view of context sensitivity of transcription within *E. coli* we undertook a transposon mutagenesis approach to randomly insert a transcription reporter cassette throughout the genome. This reporter cassette has expression of *emGFP* driven by a weak promoter (p1) and has transcriptional terminators both upstream and downstream of *emGFP* to prevent unwanted transcriptional read-through into our reporter gene from flanking genomic regions (Fig. 1). We confirmed that *emGFP* transcription is indeed derived from the weak promoter and not from read-through transcription using qPCR (material methods and Figure 7). The genomic locations for the transposon insertion sites were identified by sequencing. The final library consists of 209 strains that harbor the *emGFP* reporter cassette at unique locations covering the entire genome.

To compare the transcriptional impact that two different nucleoid structures would exert upon our reporter cassette we evaluated *emGFP* transcription levels when cells were grown in media containing either glucose or glycerol as sole carbon source [40]. The results were plotted with respect to the genomic location where the cassette inserted (Fig. 1). The results show that there is a large difference in expression profiles for cells grown in the different media. Though the averaged expression levels for each set is similar (0.0713 transcripts per cell (TPC) glucose and 0.0707 TPC glycerol) the difference between the lowest and highest expression values is 161-fold for glucose and 102-fold for glycerol. The sensitivity of a specific insertion site to context sensitivity under these two growth conditions can be further illustrated by comparing the expression values for each strain as a ratio (Glycerol TPC/Glucose TPC). We find that there is a 1378 fold difference between the highest and lowest ratios. This data suggests that certain insertion sites are more sensitive to the local context than others. We were unable to identify any correlation between our expression patterns and the macrodomains. Altogether, these results show that inserting the same reporter cassette into different genomic locations has a dramatic influence on expression and that the genomic expression patterns significantly differ from one growth condition to another and from one insertion site to another. Previous studies have only tested the influence of genomic location under a single condition and were unable to elucidate the dynamic nature of chromosome architecture and its impact on expression [1, 2].

To obtain a more detailed map of context sensitivity we inserted our reporter cassette into several loci within the genome (17 positions in the Ter region and 8 positions in the Ori). The insertion points were rationally curated to be non-mutagenic (inserted between transcription units and known regulatory features). In these regions, TPC were lower for the strains grown in glucose compared to glycerol (mean TPC are 0.043 +/- 0.019 and 0.117 +/- 0.082, respectively) and there is a lower expression variability between the strains when grown in glucose (up to 3-fold difference) versus glycerol (up to a 5.2-fold difference) media. This finding further highlights the extreme importance of position in expression and shows that the variation in expression is obvious even at positions that are very close (2-3 kb apart). The results additionally show that the variability in expression is not due to mutagenic effects caused by the transposon insertions.

**Example 3. Transcription in a protein-bound DNA loop is protected against context sensitivity**

It was decided to evaluate the impact that DNA looping has on context sensitivity. We used lambda cl protein to incorporate our reporter cassette within a small DNA loop *in vivo* and quantified *emGFP* expression (Fig. 2). Previously it has been shown that lambda cl efficiently binds to specific DNA sequences ($O_R$ & $O_L$, Fig. 1 and 2) and self-dimerizes to form a protein-bound DNA loop *in vivo* [41-44]. To express lambda cl we engineered a strain that has the gene under the transcriptional control of the rhamnose inducible promoter in the *E. coli* genome (Materials & Methods). We confirmed that this strain expressed lambda cl when rhamnose was added to the medium using Western blot analysis. We tested the impact that a DNA loop has on 16 of the non-mutagenic insertion sites (eight in the Ori region and eight in the Ter region)(Fig. 2). These strains were constructed by transferring the reporter cassettes into the lambda cl expressing strain ($\alpha$1). Expression levels were then quantified for strains grown in a medium containing glycerol and rhamnose (to express lambda cl and form the DNA-loop).

The results show that TPC values for the DNA loop constructs average at 0.05 TPC (sd 0.01 TPC) and ranged between 0.03 to 0.06 for both the Ori and Ter regions (Fig. 2). In comparison with the un-looped configuration, the mean expression level decreased 3.9-fold and expression variability (highest TPC / lowest TPC) decreased 5-fold. This data shows that incorporation of our reporter cassette within a DNA loop homogenizes p1 expression levels to 0.05 TPC and significantly reduces variability. It also suggests that the induction of the loop isolates the reporter gene from the genomic context. Depending on the position, loop formation is associated with either an increase or decrease expression levels compared to the un-looped constructs.

To further characterize the impact that DNA looping has on expression, we quantified a series of reporter constructs in which the p1 promoter was replaced by the strong p3 promoter (Fig. 3a and b). The promoter swapping was carried out in four strains (two at the Ori and two at the Ter) (loci highlighted by stars in Fig. 2). These insertion sites were selected based on results obtained with the p1 promoter. Sites 2,185,490 and 2,244,773 were selected because loop formation strongly increased expression (> 5-fold). The insertion site 4,158,229 was selected because loop induction strongly decreased expression (> 5-fold) and site 4,046,827 was chosen because the loop did not appear to have a significant impact on expression. Expression levels for the p3 constructs were quantified for cells grown in glycerol with or without a DNA loop. In the un-looped version, we obtained an average of 0.64 TPC and a range from 0.423-0.85 TPC (Fig. 3a). This data shows that the p3 promoter strength is approximately 8-fold stronger than p1 and that the expression variability is less for the strong promoter compared to the weak promoter (2-fold for p3 and 66-fold for p1). This result suggests that weak promoters are more sensitive to the genomic context than strong promoters. In the looped p3 constructs, we obtained an average 0.4194 TPC and only a slight reduction in expression variability. (Fig. 3b). Collectively, the data shows that a protein-bound DNA loop domain can effectively isolate expression derived from the reporter cassette from the local genomic context and achieve a consistent expression profile. To determine if the size of the DNA loop influences *emGFP* expression, we increased the loop size by ~250bp by inserting a fragment of neutral DNA downstream of the reporter gene. The results show that the expression values obtained from this construct are similar to the original loop construct, suggesting that in small loops (at least ≤ 2 kb) the size of the DNA loop, by itself, is not influencing transcription of genes within the loop (Fig. 3c).

**Example 4. Reducing the positive supercoiling buildup (PSB) in DNA loops increases expression levels**

Considering the results that show a DNA loop isolates gene expression from context sensitivity, we hypothesized that factors internal to the protein-bound DNA loop must influence expression of genes within the loop. We decided to test the twin supercoiling domain model for transcriptional regulation [7-9] and define the role that positive supercoiling buildup (PSB) has on expression within a DNA loop. DNA gyrase catalyzes the ATP-dependent negative supercoiling of DNA and relaxes positive supercoils introduced by transcription [25, 27, 28]. To test for PSB in loops, we used a DNA sequence of Mu-prophage that *E. coli* DNA gyrase recognizes efficiently to relax supercoiling [45]. This GRS sequence was inserted upstream and downstream of the p3 expressed *emGFP* from p3 within the loop (Fig. 3d, e). These synthetic constructs were introduced into the four genomic loci (Fig. 3).

Comparing the p3-emGFP loop construct (Fig. 3b) to the two p3-emGFP loops constructs that have the GRS (Fig. 3d, e), we observe that expression is increased from 20 to 60% in the GRS variants. We observe an increase in *emGFP* expression when the GRS is placed either upstream or downstream the reporter gene. This result demonstrates that reduction of PSB accumulation inside the loop facilitates higher expression levels.

**Example 5. Transcription levels for two genes within a DNA loop depends on their relative orientation and promoter strength**

Within natural genomes it can be assumed that multiple promoters would be present within a single protein-bound DNA loop domain. How different transcription units impact each other when expressed on the chromosome remains to be elucidated. To test the impact that a second transcribed gene within a DNA loop has on expression levels, we engineered a defined series of genetic constructs and inserted these into two different genomic locations (Ori and Ter regions) in the strain expressing lambda cl. We inserted a second reporter gene (*mCherry*) into the DNA loop in three different configurations (Fig. 4, top panel): (a) Tandem, transcription is co-oriented, (b) Convergent, the two genes transcribe into each other, (c) Divergent, transcription proceeds away from each other in opposite directions. These constructs have *mCherry* expressed from the weak p1 or strong p10 promoter and *emGFP* expressed from the strong p3 promoter. Both genes are directly followed by a strong terminator to prevent read-through transcription and transcription fork collisions.

When compared to the single *emGFP* loop construct (Fig. 3b), the insertion of p1-*mCherry* doubled *emGFP* expression in the tandem and convergent constructs while it had no effect on the divergent configuration (Fig. 4a-c and p1 bargraphs). In these constructs, expression of *mCherry* from the weak p1 promoter was very low. To define how a strong promoter within the DNA loop impacts expression we swapped the promoter for *mCherry* (weak p1 with the strong p10 promoter) and quantified expression for both genes by qPCR (Fig. 4a-c and p10 bargraphs). Comparing the *emGFP* expression profiles for the p1 and p10 constructs we see that the use of a strong promoter for *mCherry* doubles *emGFP* expression levels for the tandem and divergently oriented genes and slightly decreases expression in the convergent (Fig. 4 p1 and p10 bargraphs). Expression of *mCherry* in the convergent orientation is 2-fold lower compared to tandem and divergent.

Overall, our results show that expression levels are higher in loops containing 2 strong promoters. They additionally show that, for each construct, we obtain similar expression levels when the cassette is inserted into either the Ori or the Ter regions. This finding is consistent with our previous results that show a DNA loop can isolate gene expression from the local context, suggesting that larger DNA loops are insulated as well.

## Example 6. PSB impacts expression levels for both genes within a DNA loop

To test the impact that PSB has on expression, GRS was inserted between the two transcribed genes and mRNA levels were compared to the constructs that did not have the GRS site (Fig. 4d-f and bargraphs P10(GRS)). Insertion of GRS increased *emGFP* expression in all orientations (3 to 7-fold) and increased *mCherry* expression (2-3 fold) in the tandem and convergent configurations, while having no effect on *mCherry* expression in the divergent construct. This result shows that PSB strongly inhibits expression in loops containing two genes. The tandem oriented construct with the GRS produced the highest expression levels for both genes.

## Example 7. DNA loop formation and isolating transcription from the genomic context

Lambda cl is a highly characterized DNA-loop forming protein. The capacity of this protein to tether distant regions is provided by its ability to bind to DNA operators and to self-oligomerize. In the tetrameric form, lambda cl binds operator regions ($O_L$ and $O_R$). The interaction between the two tetramers forms an octamer and this results in the formation a DNA-loop.

To confirm that DNA loop formation and not solely DNA binding is required for transcriptionally isolating gene expression from the genomic context, we engineered 3 strains, each expressing a different mutant of lambda cl protein (P158T ; Y210H ; S228R). These mutants have been well-characterized previously [46]. Two mutations impact the oligomerization ability of lambda cl (P158T ; S228R) and the third reduces the capacity of lambda cl to bind adjacent operator sites (Y210H). Briefly, these mutations allow lambda cl to bind DNA but prevent the formation of DNA loops.

The (p3-*emGFP*)-GRS-(p10-mCherry) construct was inserted into strains expressing each of the 3 lambda cl mutants. The results show that expression levels for both *emGFP* and *mCherry* in strains where the lambda cl mutation is present is similar to expression when lambda cl is absent from the strain (Figure 8). These results confirm that the formation of a DNA loop is required for transcriptional insulation from the genomic context.

## Example 8. DNA looping and PSB are often used to control gene expression

Further support for our findings comes from the data sets described in Kroner [48]. In this work the LRP regulon [47] was analyzed. LRP is a well-characterized transcription factor that is expressed at different levels during a growth curve [48]. LRP expression is very low during exponential growth and increases significantly at the transition phase and then goes down slightly in stationary phase. LRP protein has been shown to be capable of bridging distant segments of DNA to form DNA loops in the cell [49]. The authors of [48] have done a quantitative time course analysis of *E. coli,* by LRP Chip-seq and RNA-seq in different nutrient conditions. Mapping their Chip-seq and RNA-seq data to the *E. coli* genome we have identified several genomic regions where the DNA binding patterns for LRP along with the corresponding transcriptional responses are similar to what we have shown in our work. We thus constructed a detailed map for a 40 kb genomic region to present some of the data (not shown). Strong LRP Chip-seq signals correspond to protein-bound domains and intergenic regions are assumed to form DNA loops. Based upon the levels of LRP bound to genomic DNA, we predict that a series of DNA-loops would be formed in the 40 kb region (loci 984kb to 989,5kb). The data shows that there is a high level of expression for these genes in logarithmic phase when the Chip-seq signals are low. During transition and stationary phase, the expression is significantly reduced corresponding to the higher Chip-seq reads flanking the gene and the predicted formation of protein-bound DNA loop domains. We detected 62 loci where a single gene was trapped between 2 strong LRP binding sites, among them ~90% presented reduced (or completely silenced) gene expression when Chip-seq signals increased from log to stationary phase. This data is remarkably similar to what we obtained in Fig. 2 when we incorporated the *emGFP* reporter cassette into a DNA loop. In this set, we expect that PSB would increase within the protein-bound loop domain and inhibit transcription of the corresponding gene.

Another interesting observation was made when we searched for genes in the convergent orientation. In multiple convergent gene loci we observed an increase in expression for one gene resulted in a decreased expression for the second gene, include (*smrA* and *dgcM*), (*yhjE* and *yhjG*), (*eco* and *mqo*), and (*ytfK* and *ytfL*).

Overall, this data for LRP supports our findings about PSB and the orientation of genes transcribed within predicted protein-bound DNA loops. They also suggest that other DNA binding proteins could potentially use DNA looping and PSB to modulate gene expression in response to growth conditions.

## Example 9. Summary and Discussion

Our work highlights new insights into the mechanisms of epigenetic regulation in bacteria. As a first step, we obtained an overview of the genomic landscape for this phenomena using transposon mutagenesis. Previously, different groups have taken similar approaches, where they quantitated expression levels of a reporter gene that was placed at different genomic locations. All these studies quantitated expression levels for strains grown in a single growth condition, providing a static snapshot of expression variability. In our study, we evaluated the reporter library in two growth conditions where it has been previously shown that the nucleoid architecture is drastically different. This gave us the unprecedented opportunity to obtain a dynamic view of how two different chromosomal conformations influence the expression of the exact same reporter construct genome-wide. Additionally, previous studies often used strong promoters to drive expression of the reporter gene. We demonstrate that a gene expressed from a weak promoter is more sensitive to the influence of its genomic context. Other studies have used fluorescence to track transcriptional responses. Though this is an easy way to obtain expression data, we believe that by quantitating the protein end-product, our results could be misleading due to other factors that influence final protein levels (post-transcriptional regulation, translational and post-translational regulation, and maturation of the fluorophore). For this reason, we used quantitative RT-PCR to determine expression levels, one of the most accurate methods for mRNA quantification.

From the data, it is obvious that genomic position has a dramatic influence on gene expression and that these expression profiles can differ significantly when the genome architecture is modified (Fig. 1). The two different growth conditions we used have previously been shown to result in different Ori/Ter ratios [50]. This would increase the copy numbers for genes close to the Ori, resulting in higher levels of transcription at the Ori region (gene dosage effect) [51]. Our data from the transposon library does not show this global higher skew for transcription levels at the Ori region as would be expected for a gene dosage effect. It is possible that the Ori:Ter ratio difference is not significantly different under the growth conditions tested (we isolate total RNA at an OD600=0.5).

We wanted to define the scale of context sensitivity and see if regions that are in close proximity on the genome are impacted in a similar way. Here we constructed a fine-tune map of promoter sensitivity by inserting our reporter cassette into several positions within a 100 kb region in the Ter domain (Fig. 1). In this region we observed a large difference in expression levels and variability between the glucose and glycerol medium. Within this domain there are two operons that are strongly induced in glycerol growth medium. It is possible that the strong recruitment of RNA polymerase to the region during growth in glycerol medium results in an increased localized concentration of RNA polymerase and the higher overall expression levels for strains in glycerol conditions. Increases in local concentration have been proposed to be a basic principle of transcriptional control [52]. This has been shown to lead to cooperative or competitive interactions between regulatory molecules.

We calculated the ratio of TPC glycerol:TPC glucose and plotted this value at each genomic insertion site. Five out of the six insertion points with the highest ratio had the reporter cassette inserted in a tandem orientation with respect to the neighboring genes. This finding correlates with our results showing that the tandem orientation for two genes in a DNA loop gave the highest expression level. Overall, this fine-tune mapping of context sensitivity using our reporter cassette demonstrates that even when two genomic insertion sites are close together on the genome, they can be impacted quite differently by context sensitivity.

To understand the impact that topological domains have on context sensitivity, we used lambda cl protein to incorporate *emGFP* within a DNA loop. We found that the highly variable *emGFP* expression levels were homogenized (~0,05 TPC in 16 loci) when incorporated within a DNA loop, significantly reducing strain-to-strain variability. The control experiments using lambda cl mutants demonstrate that this effect depends on DNA loop formation. This data supports previous findings that have shown DNA loops can form topological domains. The use of DNA loops can be extremely beneficial for Synthetic Biology projects to; (i) Reduce transcriptional variability in several genomic locations and (ii) Effectively isolate the expression of synthetic circuits from the local genomic context to achieve a consistent / predictable expression profile. We additionally demonstrate that a DNA loop can effectively isolate expression derived from a strong p3 promoter for all genomic positions tested. However, in the looped version the expression levels are often lower than in the un-looped version. We demonstrate that this is due to PSB accumulation in the DNA loop by introducing GRS inside the DNA loop. We were able to reduce accumulation of PSB and increase *emGFP* expression up to 700% compared to the same construct without GRS. These observations strongly suggest that PSB is a major actor of the regulatory mechanism controlling expression levels for genes incorporated within protein-bound DNA loops. Thus, any effector that changes supercoiling levels can impact gene transcription.

To further characterize expression within a protein-bound DNA loop we introduced a second gene expressed by promoters of different strengths in different configurations. The tandem gene orientation gave the highest expression levels for both reporter genes. Indeed, according to the twin-supercoiled domain model we would expect the positive supercoil induced by the transcription of *emGFP* to be countered by the negative supercoil induced by the transcription of *mCherry*. A decrease of topological constraints in the intergenic region could be beneficial for the expression of both genes. For *emGFP,* RNA polymerase initiation is subject to less constraints during the transcription, for *mCherry,* the recruitment of the enzyme or the transition to open complex are facilitated. We additionally observed that when mCherry is expressed by the weak p1 promoter, we obtained extremely low expression values in both the convergent and divergent constructs which shows that weak promoters are very sensitive genomic context and can be "overwhelmed" by transcription derived from the strong p3 promoter.

In the convergent orientation, when comparing data from the weak and the strong promoter for *mCherry,* we see that an increase of expression of *mCherry* results in a decrease of expression for *emGFP*. This configuration would be predicted to induce the highest accumulation of positive supercoil in the intergenic region. The results obtained confirm the negative impact of PSB on gene expression. Interestingly, the ~0,2 TPC increase of *mCherry* results in ~0,16 TPC decrease of *emGFP*. This observation suggests that transcription levels are directly proportional to positive supercoiling levels in a localized region. It appears that a limited quantity of PSB is tolerated between these two genes and the total transcripts for this configuration is partitioned between the two genes based upon relative promoter strength. In a recent work, Bryant *et al.* [1] also observed the mutual negative impact of convergent transcriptional units. Overall, these observations strongly suggest that expression of convergent genes impede each other. Another interesting finding is that *emGFP* expression actually increases in the tandem and divergent constructs even though the *emGFP* promoter was not changed in the data sets that have mCherry expressed by p1 and p10. We hypothesized that this could be due to increases in the local concentration of RNA polymerase recruited by the strong promoter.

To test the impact that PSB has on constructs that have two strong promoters, a GRS site was inserted between the two genes. This had a positive impact on expression for all three *emGFP* constructs (3.5-fold increase in tandem, 7-fold in convergent, 2.5-fold in divergent). The GRS also significantly improved expression of *mCherry* in the tandem construct (3-fold) and in the convergent construct (2-fold) but only had a moderate impact on levels for the divergent construct. For *emGFP* the expression trend is, tandem > convergent > divergent. This pattern is different than what was obtained by Yeung et al. [4] where they performed a study evaluating the orientation of transcribed genes on a plasmid. We believe that this can be explained by the fact that a plasmid is not tethered and is free to diffuse supercoiling to the vector backbone region and thus absorb some of the torsion that is created. Which in turn, has a different impact on transcription. We postulate that tandem organization is the optimal configuration for high gene expression. Studies made on natural organization of genes on the bacterial chromosome corroborate our observations [53-55].

Recent work characterizing LRP has provided strong evidence supporting the mechanisms we have uncovered. LRP is a transcriptional regulator that is expressed at higher levels in the transition and stationary growth phase. LRP has been shown to form DNA loops *in vivo,* in the same way as lambda cl can. The protein structure, DNA binding and regulation by LRP has been well-studied [49, 56, 57]. Chip-seq and RNA-seq data of LRP [47] characterized the role of LRP on gene expression. Evaluating these data sets, we discover a difference in expression for many genes that correlates with the accumulation of LRP in transition and stationary phase. For example, we have found that single genes trapped between two LRP binding sites (which would be predicted to induce a DNA loop), present decrease or silencing of expression in ~90% of the cases (55 of 62 genes). Overall, these observations strongly suggest that LRP regulates gene expression by forming small DNA-loops in the transition between log and stationary phases.

One feature of LRP is it's involvement in modulating the expression of genes important for the transition from rapid to slowed growth. Among this family of regulated genes is the ribosomal RNA (rRNA). The promoters for the seven rRNA operons in *E. coli* are among the strongest in the cell. These are regulated by a complex mechanism involving several proteins and small molecules. LRP has been shown to be involved with the regulation of these promoters but the exact mechanism has not been well-defined [58-60]. DNA footprints using LRP suggest that looping or wrapping of DNA could be leading to a repressosome structure [59]. In the LRP Chip-seq data, a peak can be observed in the 5' untranslated region directly upstream for 6 of the 7 rRNAs. Kroner *et. al.* have stated that LRP often binds to promoters in a poised position and has no regulatory activity. They continue to propose that this enables combinatorial interactions with other regulators. We propose that this poised binding in the rRNAs has a structural role related to DNA looping. In the stationary phase Chip-seq data, there are a series of peaks upstream of the promoter that could form DNA loops that would isolate the rRNA promoter from the downstream transcribed region. It has additionally been demonstrated that rRNA operons are in close physical proximity within the cell and that this is independent of their transcription activities [61]. The bridging of distant DNA regions by LRP could potentially explain this finding.

LRP is just one of many proteins that are able to form DNA loops. Similar studies that use Chip-seq and RNA-seq data will prove useful for further defining the how the chromosome architecture impacts expression. Work done previously by Adhya's group characterizing GalR additionally supports our findings [62]. GalR forms DNA loops *in vivo.* Using mutant strains, microarrays, CHIP-seq and a bioinformatic approach, they concluded that GalR indirectly regulates transcription by inducing large-scale restructuring of the chromosome. LRP and GalR are just two of many proteins that are able to form DNA loops. Similar studies that use Chip-seq and RNA-seq data will prove useful for further defining the how the chromosome architecture impacts expression.

This study has permitted us to formulate a comprehensive model that explains the observed position effects on promoter activity in *E. coli.* Our data support a model of an epigenetic mechanism that is defined by the dynamic local DNA architecture (protein-bound DNA domains) and transcriptionally induced positive supercoiling buildup (Fig. 6). Our work quantifying the impact that altering the chromosomal conformation has on expression levels demonstrates the genome-wide scale of this regulatory mechanism. It has been well-documented how different proteins shape the genomic architecture and that the level and activity of these proteins change dynamically with growth phase and environmental conditions. The differential binding of these proteins creates different DNA loops / genomic architecture. We propose that the formation of different protein-bound DNA domains cause genes to be transcribed in different orientations and configurations, thus epigenetically impacting their expression as we have demonstrated within this work. Our model explains how hundreds of genes can either be turned on or off within less than two minutes after the cell is exposed to an environmental stress or stimulus. This is far less time than is required to transcribe and translate new proteins to respond the environmental signal. Certain post-translational modifications (phosphorylation, acetylation, etc.) have been shown to impact DNA binding proteins, altering their capacity to bind DNA. These modifications have also been shown to occur very rapidly within the cell, suggesting that post-translational modifications may additionally play a role in this dynamic regulatory event. Our model tightly correlates with the recent findings from L. Serrano's lab [63]. In this work they have reported that a large part of transcriptional regulation is determined by non-canonical factors such as DNA supercoiling and genome organization. The diagram of a simplified genomic region that contains sequences for different DNA binding proteins illustrates how binding by different proteins that are capable of self-oligomerization in this region would result in several alternate configurations depending upon the relative level and activity (Fig. 6). Based upon this work we would expect that these different DNA loop conformations would produce very different levels for the "B" transcript. We are now applying these basic concepts to the design of complex genetic programs and synthetic genomes that we are currently engineering within our labs.

## Conclusions

This work has uncovered the underlying mechanisms that govern epigenetic regulation in bacteria. We have shown that context sensitivity is due to inserting a heterologous cassette into an uncharacterized protein-bound DNA domain that naturally contains multiple transcription units that impact the expression of each other. The observed variability in expression levels is driven by the local DNA topology, genome layout, promoter strength, orientation of transcription units, and supercoiling. We have additionally demonstrated that a synthetic circuit can be effectively isolated from the local genomic context by incorporating it within a protein-bound DNA loop structure. The molecular mechanisms we have elucidated will lead to; (i) New fundamental discoveries and advances in Synthetic Biology, (ii) identification of new targets for antimicrobial compounds, and (iii) The design/engineering of synthetic genomes.

## Summary

Expression from engineered circuits can vary significantly when inserted into different genomic locations. This unpredictable performance complicates the implementation of larger genetic programs and the engineering of synthetic genomes. Currently, it is not known what causes position effects on promoter activity.

A library of strains that have a reporter cassette randomly inserted into the *Escherichia coli* genome was constructed. Expression of the library in two growth conditions that induce different chromosomal conformations was quantified and it was shown that transcript levels varied significantly. Incorporating this cassette within a protein-bound DNA loop reduced expression variability 31-fold. Testing a series of synthetic DNA loops (encoding different genetic layouts) inserted into different genomic locations, the impact that gene orientation and positive supercoiling buildup has on gene expression was defined. Evaluating a multi-Omic dataset, similar patterns of mRNA expression correlated with DNA loop formation were found.

It is presented a unifying model that explains the underlying molecular mechanism responsible for epigenetic regulation in bacteria. The model provides an explanation for how bacteria control large families of genes in a rapid and coherent response to environmental stresses. The nucleoid architecture is dynamically remodeled through the activity of differentially expressed DNA binding proteins. Alternate chromosomal conformations induce different DNA looping structures and expression of genes within these looped domains are sensitive to transcriptionally induced positive supercoiling buildup, promoter strength and gene orientation. Expression from genes in a genome is influenced by a combination of genome layout, DNA binding proteins, transcription within protein-bound DNA loops and supercoiling.

## References

1. Bryant JA, Sellars LE, Busby SJW, Lee DJ. Chromosome position effects on gene expression in Escherichia coli K-12. Nucleic Acids Res. 2014;42:11383-92.
2. Scholz SA, Diao R, Wolfe MB, Fivenson EM, Lin XN, Freddolino PL. High-Resolution Mapping of the Escherichia coli Chromosome Reveals Positions of High and Low Transcription. Cell Syst. 2019;8:212-225.e9.
3. Kolkhof P, Müller-Hill B. Lambda cl Repressor Mutants Altered in Transcriptional Activation. J Mol Biol. 1994;242:23-36.
4. Yeung E, Dy AJ, Martin KB, Ng AH, Del Vecchio D, Beck JL, et al. Biophysical Constraints Arising from Compositional Context in Synthetic Gene Networks. Cell Syst. 2017;5:11-24.e12.
5. Peter BJ, Arsuaga J, Breier AM, Khodursky AB, Brown PO, Cozzarelli NR. Genomic transcriptional response to loss of chromosomal supercoiling in Escherichia coli. Genome Biol. 2004;5:R87.
6. Postow L, Hardy CD, Arsuaga J, Cozzarelli NR. Topological domain structure of the Escherichia coli chromosome. Genes Dev. 2004;18:1766-79.
7. Deng S, Stein RA, Higgins NP. Organization of supercoil domains and their reorganization by transcription. Mol Microbiol. 2005;57:1511-21.
8. Liu LF, Wang JC. Supercoiling of the DNA template during transcription. Proc Natl Acad Sci USA. 1987;84:7024-7.
9. Ma J, Bai L, Wang MD. Transcription under torsion. Science. 2013;340:1580-3.
10. Ma J, Wang M. Interplay between DNA supercoiling and transcription elongation. Transcription. 2014;5:e28636.
11. Taniguchi Y, Choi PJ, Li G-W, Chen H, Babu M, Hearn J, et al. Quantifying E. coli proteome and transcriptome with single-molecule sensitivity in single cells. Science. 2010;329:533-8.
12. Wagner R. Transcription regulation in prokaryotes. Oxford; New York: Oxford University Press; 2000.
13. Chong S, Chen C, Ge H, Xie XS. Mechanism of Transcriptional Bursting in Bacteria. Cell. 2014;158:314-26.
14. El Houdaigui B, Forquet R, Hindré T, Schneider D, Nasser W, Reverchon S, et al. Bacterial genome architecture shapes global transcriptional regulation by DNA supercoiling. Nucleic Acids Res. 2019;47:5648-57.
15. Dorman CJ. Genome architecture and global gene regulation in bacteria: making progress towards a unified model? Nat Rev Microbiol. 2013;11:349-55.
16. Jin DJ, Cagliero C, Zhou YN. Growth rate regulation in Escherichia coli. FEMS Microbiol Rev. 2012;36:269-87.
17. Jin DJ, Cagliero C, Martin CM, Izard J, Zhou YN. The dynamic nature and territory of transcriptional machinery in the bacterial chromosome. Front Microbiol. 2015;6:497.
18. Cournac A, Plumbridge J. DNA looping in prokaryotes: experimental and theoretical approaches. J Bacteriol. 2013;195:1109-19.
19. Müller-Hill B. The lac Operon: a short history of a genetic paradigm /. Berlin; Walter de Gruyter,; 1996.
20. Vilar JMG, Leibler S. DNA looping and physical constraints on transcription regulation. J Mol Biol. 2003;331:981-9.
21. Vinograd J, Lebowitz J, Radloff R, Watson R, Laipis P. The twisted circular form of polyoma viral DNA. Proc Natl Acad Sci. 1965;53:1104-11.
22. Strick TR, Allemand JF, Bensimon D, Bensimon A, Croquette V. The elasticity of a single supercoiled DNA molecule. Science. 1996;271:1835-7.
23. Lepage T, Képès F, Junier I. Thermodynamics of long supercoiled molecules: insights from highly efficient Monte Carlo simulations. Biophys J. 2015;109:135-43.
24. Kouzine F, Gupta A, Baranello L, Wojtowicz D, Benaissa K, Liu J, et al. Transcription dependent dynamic supercoiling is a short-range genomic force. Nat Struct Mol Biol. 2013;20:396-403.
25. Palma CSD, Kandavalli V, Bahrudeen MNM, Minoia M, Chauhan V, Dash S, et al. Dissecting the in vivo dynamics of transcription locking due to positive supercoiling buildup. Biochim Biophys Acta Gene Regul Mech. 2020;1863:194515.
26. Wang JC. Moving one DNA double helix through another by a type II DNA topoisomerase: the story of a simple molecular machine. Q Rev Biophys. 1998;31:107-44.
27. Lal A, Dhar A, Trostel A, Kouzine F, Seshasayee ASN, Adhya S. Genome scale patterns of supercoiling in a bacterial chromosome. Nat Commun. 2016;7:11055.
28. Champoux JJ. DNA Topoisomerases: Structure, Function, and Mechanism. Annu Rev Biochem. 2001;70:369-413.
29. Gellert M, Mizuuchi K, O'Dea MH, Nash HA. DNA gyrase: an enzyme that introduces superhelical turns into DNA. Proc Natl Acad Sci USA. 1976;73:3872-6.
30. Zechiedrich EL, Khodursky AB, Bachellier S, Schneider R, Chen D, Lilley DM, et al. Roles of topoisomerases in maintaining steady-state DNA supercoiling in Escherichia coli. J Biol Chem. 2000;275:8103-13.
31. Khodursky AB, Peter BJ, Schmid MB, DeRisi J, Botstein D, Brown PO, et al. Analysis of topoisomerase function in bacterial replication fork movement: Use of DNA microarrays. Proc Natl Acad Sci USA. 2000;97:9419-24.

32. Sinden RR, Pettijohn DE. Chromosomes in living Escherichia coli cells are segregated into domains of supercoiling. Proc Natl Acad Sci USA. 1981;78:224-8.

33. Kamagata K, Mano E, Ouchi K, Kanbayashi S, Johnson RC. High Free-Energy Barrier of 1D Diffusion Along DNA by Architectural DNA-Binding Proteins. J Mol Biol. 2018;430:655-67.

34. Dages S, Zhi X, Leng F. Fis protein forms DNA topological barriers to confine transcription-coupled DNA supercoiling in Escherichia coli. FEBS Lett. 2020;594:791-8.

35. Japaridze A, Yang W, Dekker C, Nasser W, Muskhelishvili G. DNA sequence-directed cooperation between nucleoid-associated proteins. preprint. Biophysics; 2020. doi:10.1101/2020.06.14.150516.

36. Higgins NP, Yang X, Fu Q, Roth JR. Surveying a supercoil domain by using the gamma delta resolution system in Salmonella typhimurium. J Bacteriol. 1996; 178:2825-35.

37. Yan Y, Ding Y, Leng F, Dunlap D, Finzi L. Protein-mediated loops in supercoiled DNA create large topological domains. Nucleic Acids Res. 2018;46:4417-24.

38. Leng F, Chen B, Dunlap DD. Dividing a supercoiled DNA molecule into two independent topological domains. Proc Natl Acad Sci USA. 2011;108:19973-8.

39. Moulin L, Rahmouni AR, Boccard F. Topological insulators inhibit diffusion of transcription-induced positive supercoils in the chromosome of Escherichia coli: Diffusion of supercolis and topogical insulators. Mol Microbiol. 2004;55:601-10.

40. Dimri GP, Rudd KE, Morgan MK, Bayat H, Ames GF. Physical mapping of repetitive extragenic palindromic sequences in Escherichia coli and phylogenetic distribution among Escherichia coli strains and other enteric bacteria. J Bacteriol. 1992; 174:4583-93.

41. Verma SC, Qian Z, Adhya SL. Architecture of the Escherichia coli nucleoid. PLOS Genet. 2019;15:e1008456.

42. Revet B, Wilcken-Bergmann B von, Bessert H, Barker A, Müller-Hill B. Four dimers of λ repressor bound to two suitably spaced pairs of λ operators form octamers and DNA loops over large distances. Curr Biol. 1999;9:151-4.

43. Dodd IB, Perkins AJ, Tsemitsidis D, Egan JB. Octamerization of λ CI repressor is needed for effective repression of P RM and efficient switching from lysogeny. Genes Dev. 2001;15:3013-22.

44. Dodd IB, Shearwin KE, Perkins AJ, Burr T, Hochschild A, Egan JB. Cooperativity in long-range gene regulation by the lambda CI repressor. Genes Dev. 2004;18:344-54.

45. Ding Y, Manzo C, Fulcrand G, Leng F, Dunlap D, Finzi L. DNA supercoiling: a regulatory signal for the λ repressor. Proc Natl Acad Sci USA. 2014;111:15402-7.

46. Oram M, Pato ML. Mu-like prophage strong gyrase site sequences: analysis of properties required for promoting efficient mu DNA replication. J Bacteriol. 2004; 186:4575-84.

47. Burz DS, Ackers GK. Single-site mutations in the C-terminal domain of bacteriophage lambda cl repressor alter cooperative interactions between dimers adjacently bound to OR. Biochemistry. 1994;33:8406-16.

48. Kroner GM, Wolfe MB, Freddolino PL. Escherichia coli Lrp Regulates One-Third of the Genome via Direct, Cooperative, and Indirect Routes. J Bacteriol. 2019;201.

49. Tani TH, Khodursky A, Blumenthal RM, Brown PO, Matthews RG. Adaptation to famine: a family of stationary-phase genes revealed by microarray analysis. Proc Natl Acad Sci USA. 2002;99:13471-6.

50. Chen S, Hao Z, Bieniek E, Calvo JM. Modulation of Lrp action in Escherichia coli by leucine: effects on non-specific binding of Lrp to DNA. J Mol Biol. 2001;314:1067-75.

51. Fernandez-Coll L, Maciąg-Dorszyńska M, Tailor K, Vadia S, Levin PA, Szalewska-Pałasz A, et al. The Absence of (p)ppGpp Renders Initiation of Escherichia coli Chromosomal DNA Synthesis Independent of Growth Rates. mBio. 2020.

52. Chandler MG, Pritchard RH. The effect of gene concentration and relative gene dosage on gene output in Escherichia coli. Mol Gen Genet MGG. 1975;138:127-41.

53. Oehler S, Müller-Hill B. High local concentration: a fundamental strategy of life. J Mol Biol. 2010;395:242-53.

54. Képès F, Jester BC, Lepage T, Rafiei N, Rosu B, Junier I. The layout of a bacterial genome. FEBS Lett. 2012;586:2043-8.

55. Junier I, Rivoire O. Conserved Units of Co-Expression in Bacterial Genomes: An Evolutionary Insight into Transcriptional Regulation. PLoS ONE. 2016;11. doi: 10. 1371/journal. pone. 0155740.

56. Jeong KS, Ahn J, Khodursky AB. Spatial patterns of transcriptional activity in the chromosome of Escherichia coli. Genome Biol. 2004;5:R86.

57. Tapias A, Lopez G, Ayora S. Bacillus subtilis LrpC is a sequence-independent DNA-binding and DNA-bending protein which bridges DNA. Nucleic Acids Res. 2000;28:552-9.

58. de los Rios S, Perona JJ. Structure of the Escherichia coli leucine-responsive regulatory protein Lrp reveals a novel octameric assembly. J Mol Biol. 2007;366:1589-602.

59. Pul U, Lux B, Wurm R, Wagner R. Effect of upstream curvature and transcription factors H-NS and LRP on the efficiency of Escherichia coli rRNA promoters P1 and P2 - a phasing analysis. Microbiol Read Engl. 2008;154 Pt 9:2546-58.

60. Pul U, Wurm R, Wagner R. The role of LRP and H-NS in transcription regulation: involvement of synergism, allostery and macromolecular crowding. J Mol Biol. 2007;366:900-15.

61. Pul U, Wurm R, Lux B, Meltzer M, Menzel A, Wagner R. LRP and H-NScooperative partners for transcription regulation at Escherichia coli rRNA promoters. Mol Microbiol. 2005;58:864-76.

62. Gaal T, Bratton BP, Sanchez-Vazquez P, Sliwicki A, Sliwicki K, Vegel A, et al. Colocalization of distant chromosomal loci in space in E. coli: a bacterial nucleolus. Genes Dev. 2016;30:2272-85.

63. Qian Z, Trostel A, Lewis DEA, Lee SJ, He X, Stringer AM, et al. Genome-Wide Transcriptional Regulation and Chromosome Structural Arrangement by GalR in E. coli. Front Mol Biosci. 2016;3. doi:10.3389/fmolb.2016.00074.

64. Yus E, Lloréns-Rico V, Martinez S, Gallo C, Eilers H, Blötz C, et al. Determination of the Gene Regulatory Network of a Genome-Reduced Bacterium Highlights Alternative Regulation Independent of Transcription Factors. Cell Syst. 2019;9: 143-158.e13.

65. Rossignol M, Basset A, Espéli O, Boccard F. NKBOR, a mini-Tn10-based transposon for random insertion in the chromosome of Gram-negative bacteria and the rapid recovery of sequences flanking the insertion sites in Escherichia coli. Res Microbiol. 2001;152:481-5.

66. Kuhlman TE, Cox EC. Site-specific chromosomal integration of large synthetic constructs. Nucleic Acids Res. 2010;38:e92.

67. Kuhlman TE, Cox EC. A place for everything: chromosomal integration of large constructs. Bioeng Bugs. 2010;1:296-9.

68. Keseler IM, Mackie A, Santos-Zavaleta A, Billington R, Bonavides-Martínez C, Caspi R, et al. The EcoCyc database: reflecting new knowledge about Escherichia coli K-12. Nucleic Acids Res. 2017;45 Database issue:D543-50.

69. Green MR, Sambrook J. Molecular Cloning: A Laboratory Manual (Fourth Edition). Cold Spring Harbor Laboratory Press; 2012.

70. Calculations: Converting from nanograms to copy number. Default. https://eu.idtdna.com/pages/education/decoded/article/calculations-converting-from-nanograms-to-copy-number. Accessed 23 Jun 2020.

71. Philips RM& R. » How many mRNAs are in a cell? http://book.bionumbers.org/how-many-mrnas-are-in-a-cell/. Accessed 23 Jun 2020.

## Claims

1. A cell comprising, in its genome, an expression construct comprising the sequence of a gene of interest operatively linked to elements allowing its expression in the cell, wherein the expression construct is between two DNA regions that are recognized by a DNA-binding protein that is able to bind to and bridge separate DNA regions.

2. The cell of claim 1, which is a prokaryotic cell, in particular *Escherichia coli* or *Bacillus subtilis.*

3. The cell of claim 1, which is a eukaryotic cell in particular a yeast or a mammal cell (in particular Cho cell or human cell, also list cells that are used to produce proteins in the pharmaceutical industry).

4. The cell of any one of claims 1 to 3, wherein the transgene is integrated in a natural chromosome of the cell.

5. The cell of any one of claims 1 to 3, wherein the transgene is integrated in an artificial chromosome of the cell.

6. The cell of any one of claims 1 to 5, which also comprises, into its genome, a gene coding for the DNA-binding protein able to bind to and bridge the DNA regions, operatively linked to elements allowing its expression, wherein said elements include a promoter.

7. The cell of claim 6, wherein the promoter is an inducible promoter.

8. The cell of any one of claims 1 to 8, wherein the DNA-binding protein is selected from the group consisting of lambda CI protein, galR, LRP, bivalent dCas9 complexes, Nucleoide Associated Proteins (NAPs).

9. The cell of any one of claims 1 to 9, wherein the two DNA regions are distant of between 1 and 20kb, preferably of at least 5 kb.

10. The cell of any one of claims 1 to 10, wherein the two DNA regions are identical.

11. The cell of any one of claims 1 to 11, wherein two transgenes are present between the DNA regions that are recognized by the DNA-binding protein, wherein the transgenes are in the same orientation.

**12.** A construct for transformation of a cell, comprising an expression construct comprising a promoter sequence, a gene sequence and a terminator sequence functional in the cell, wherein the expression construct is between two DNA regions that are recognized by a DNA-binding protein able to bind to and bridge these DNA regions [by self-dimerization/multimerization) so that binding of the DNA-binding protein to the DNA regions creates a DNA loop thereby isolating the expression construct from the local genomic context].

**13.** A method for obtaining the cell of any one of claims 1 to 12, comprising transforming a cell with the construct of claim 13, so as to integrate the construct within the cell genome.

**14.** A method for reducing transcriptional variability of a transgene introduced in a cell genome, wherein the transgene comprises a gene of interest operatively linked to elements allowing its expression, comprising introducing the transgene in the cell genome between two DNA regions that are recognized by the DNA-binding protein, and expressing the DNA-binding protein so that binding of the DNA-binding protein to the DNA regions creates a DNA loop thereby isolating the expression construct from the local genomic context.

**15.** A kit containing cells that have been engineered to present genomic DNA bridging regions that are recognized by a DNA-binding protein that is able to bind to and bridge these genomic DNA bridging regions, and DNA sequences between the genomic bridging regions that facilitate the insertion of heterologous sequences for transgene expression.

**16.** The kit of claim 15 wherein the DNA sequences between the genomic bridging regions include sequences of restriction enzymes.

**17.** The kit of claim 15, wherein the DNA sequences between the genomic bridging regions include sequences that are homologous to sequences of vectors used to clone the transgene and that surround the transgene.

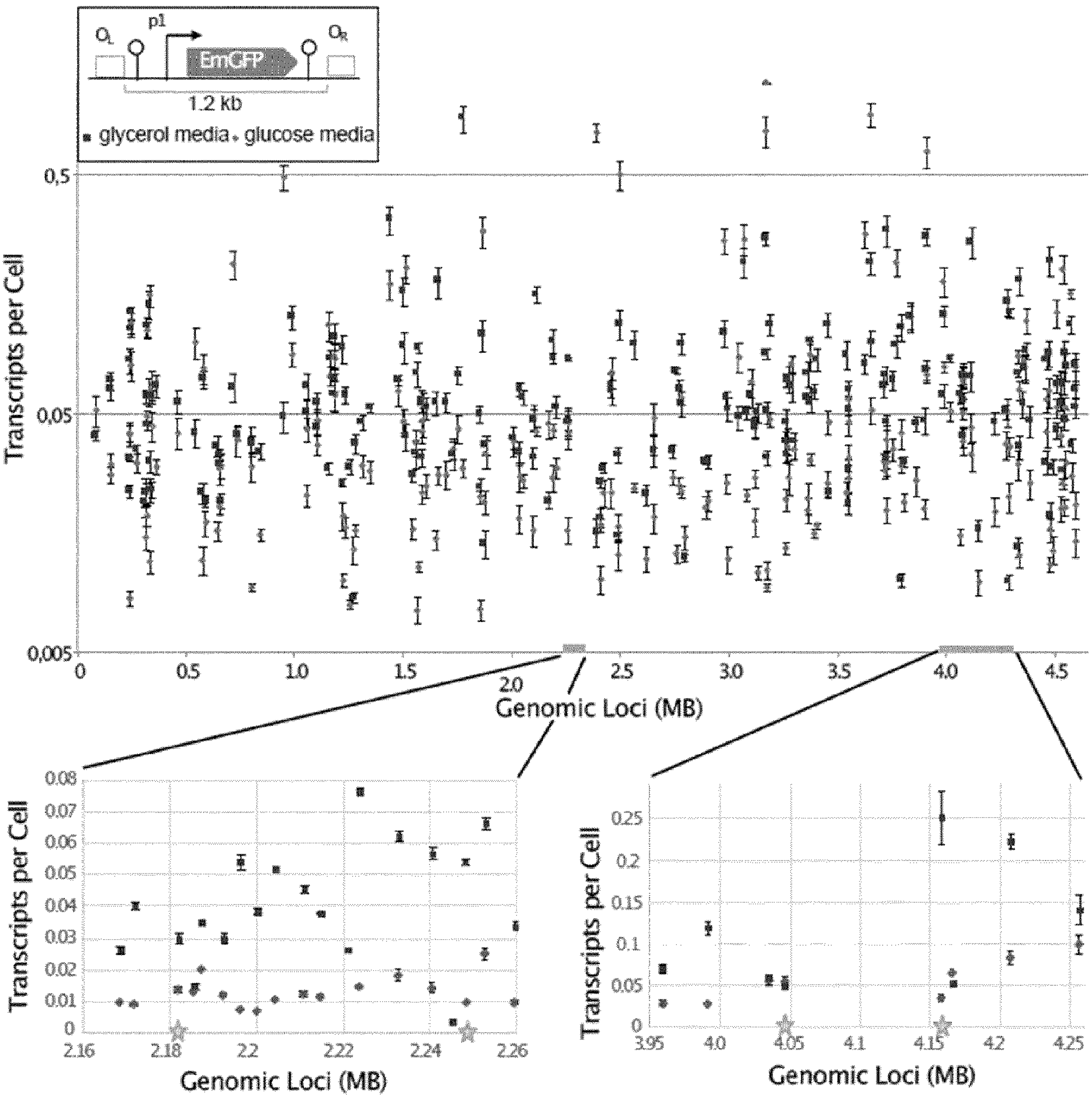
O_L
p1
EmCFP
O_R
1.2 kb
glycerol media
glucose media
Transcripts per Cell
Genomic Loci (MB)
Transcripts per Cell
Genomic Loci (MB)
Transcripts per Cell
Genomic Loci (MB)

# Figure 1

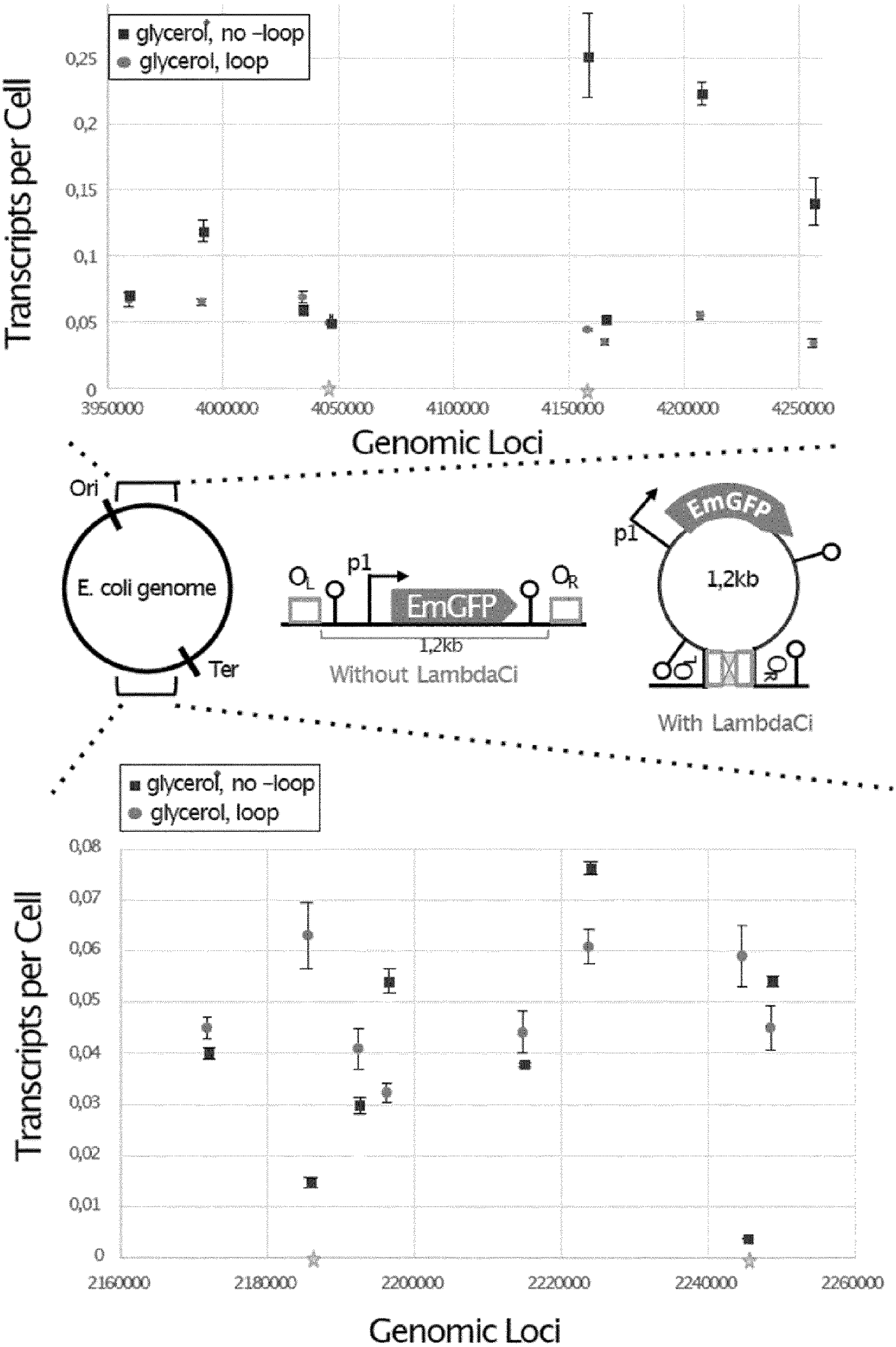
glycerol, no -loop
glycerol, loop
Transcripts per Cell
0,25
0,2
0,15
0,1
0,05
0
3950000
4000000
4050000
4100000
4150000
4200000
4250000
Genomic Loci
Ori
Ter
E. coli genome
$O_L$
p1
EmGFP
$O_R$
1,2kb
Without LambdaCi
p1
EmGFP
1,2kb
With LambdaCi
glycerol, no -loop
glycerol, loop
0,08
0,07
0,06
0,05
0,04
0,03
0,02
0,01
0
Transcripts per Cell
2160000
2180000
2200000
2220000
2240000
2260000
Genomic Loci

**Figure 2**

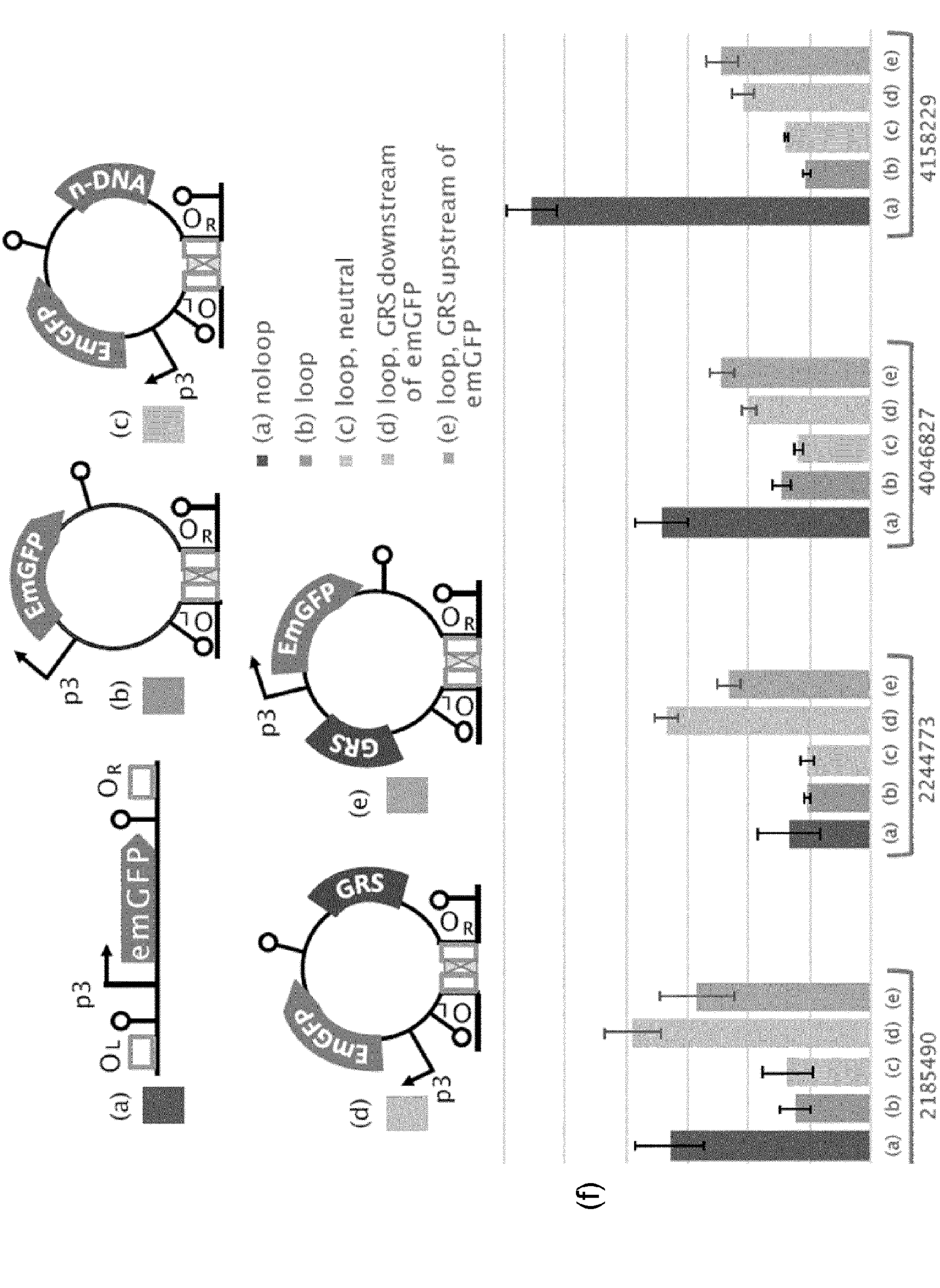
(a)
(b)
(c)
(d)
(e)
(f)
p3
emGFP
EmGFP
n-DNA
GRS
OL
OR
(a) noloop
(b) loop
(c) loop, neutral
(d) loop, GRS downstream of emGFP
(e) loop, GRS upstream of emGFP
2185490
2244773
4046827
4158229

Figure 3

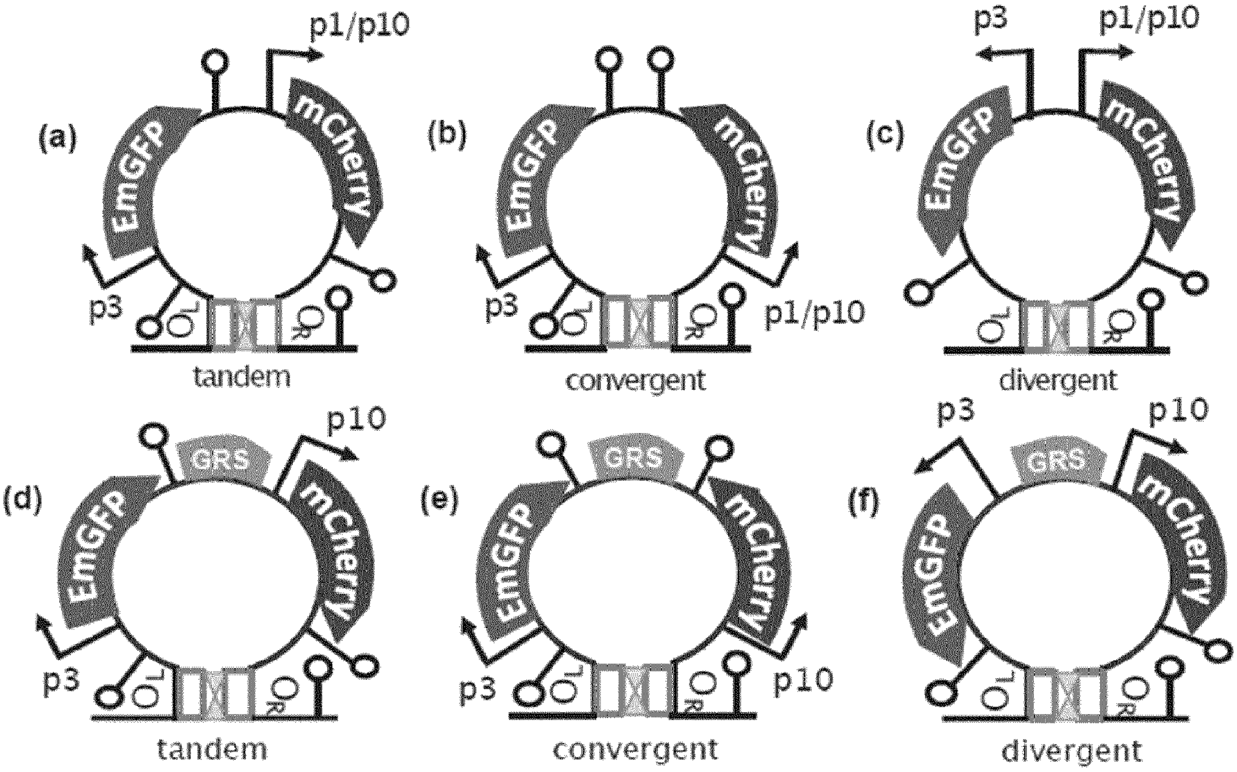
(a)
p1/p10
mCherry
EmGFP
p3
$O_L$
$O_R$
tandem
(b)
EmGFP
mCherry
p3
p1/p10
$O_L$
$O_R$
convergent
(c)
p3
p1/p10
EmGFP
mCherry
$O_L$
$O_R$
divergent
(d)
p10
GRS
EmGFP
mCherry
p3
$O_L$
$O_R$
tandem
(e)
GRS
EmGFP
mCherry
p3
p10
$O_L$
$O_R$
convergent
(f)
p3
p10
GRS
EmGFP
mCherry
$O_L$
$O_R$
divergent

A.

Figure 4

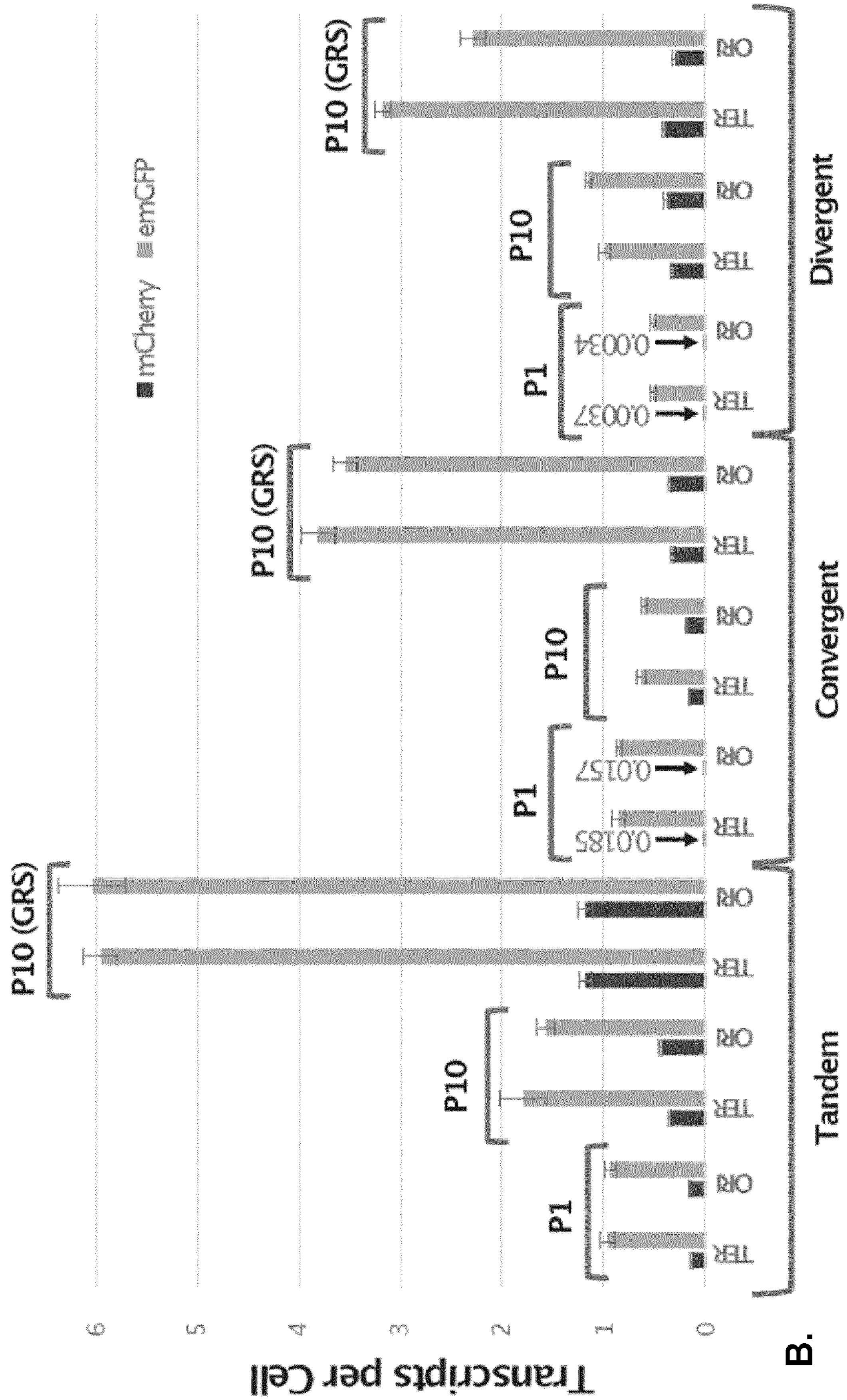


Figure 4 (continued)

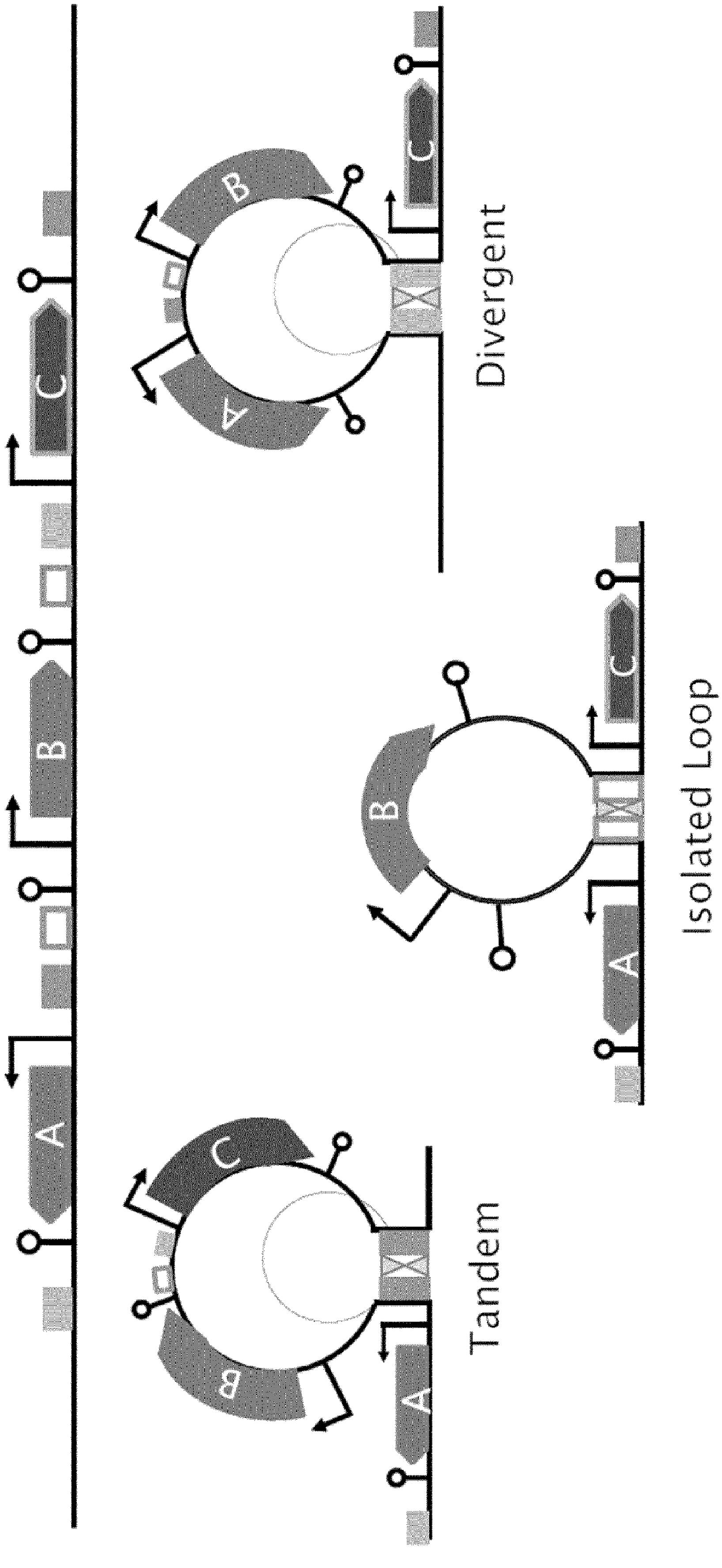


Figure 5

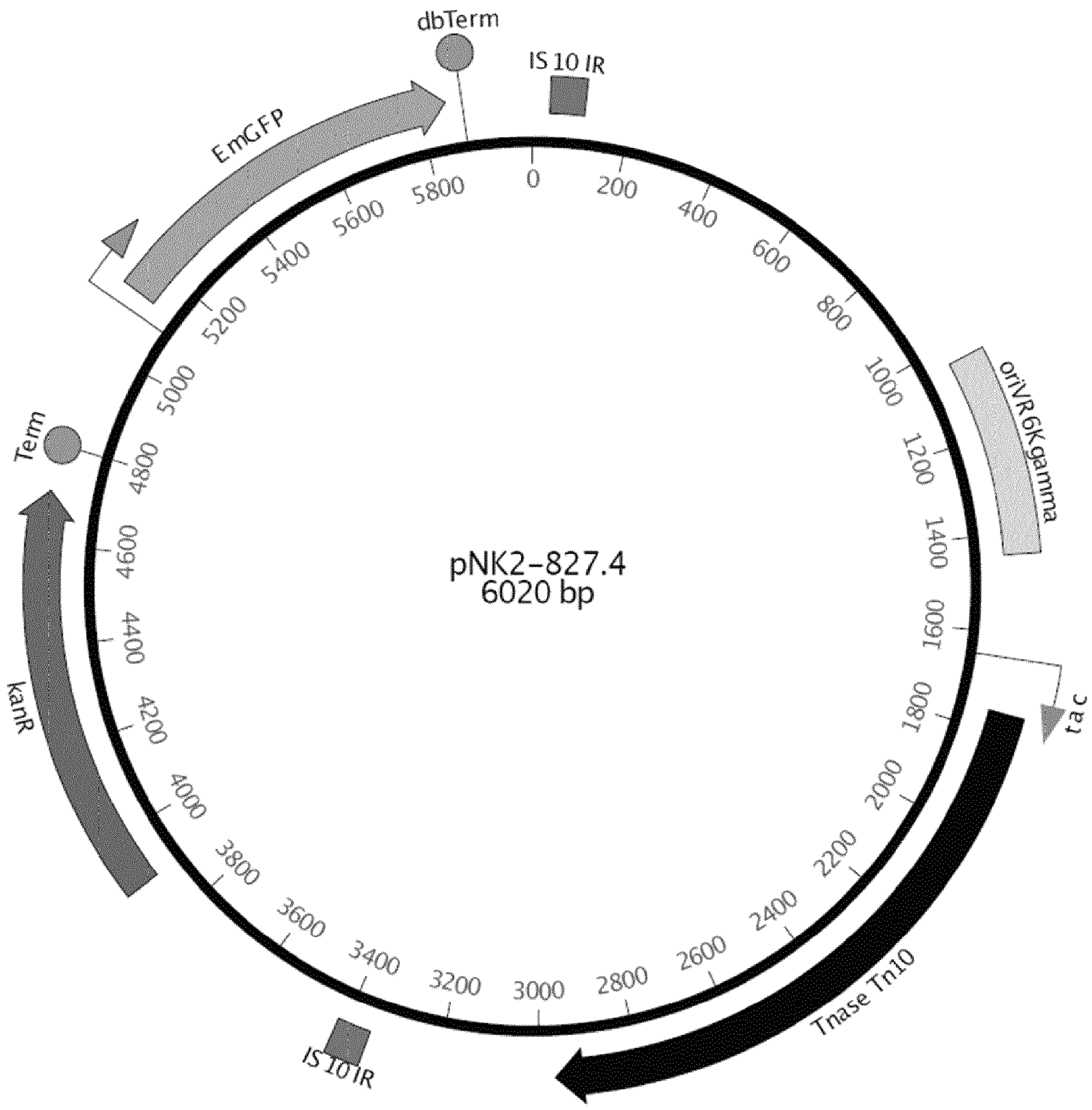
dbTerm
IS 10 IR
EmGFP
oriVR6Kgamma
Term
pNK2-827.4
6020 bp
kanR
tac
Tnase Tn10
IS 10 IR
0
200
400
600
800
1000
1200
1400
1600
1800
2000
2200
2400
2600
2800
3000
3200
3400
3600
3800
4000
4200
4400
4600
4800
5000
5200
5400
5600
5800

Figure 6

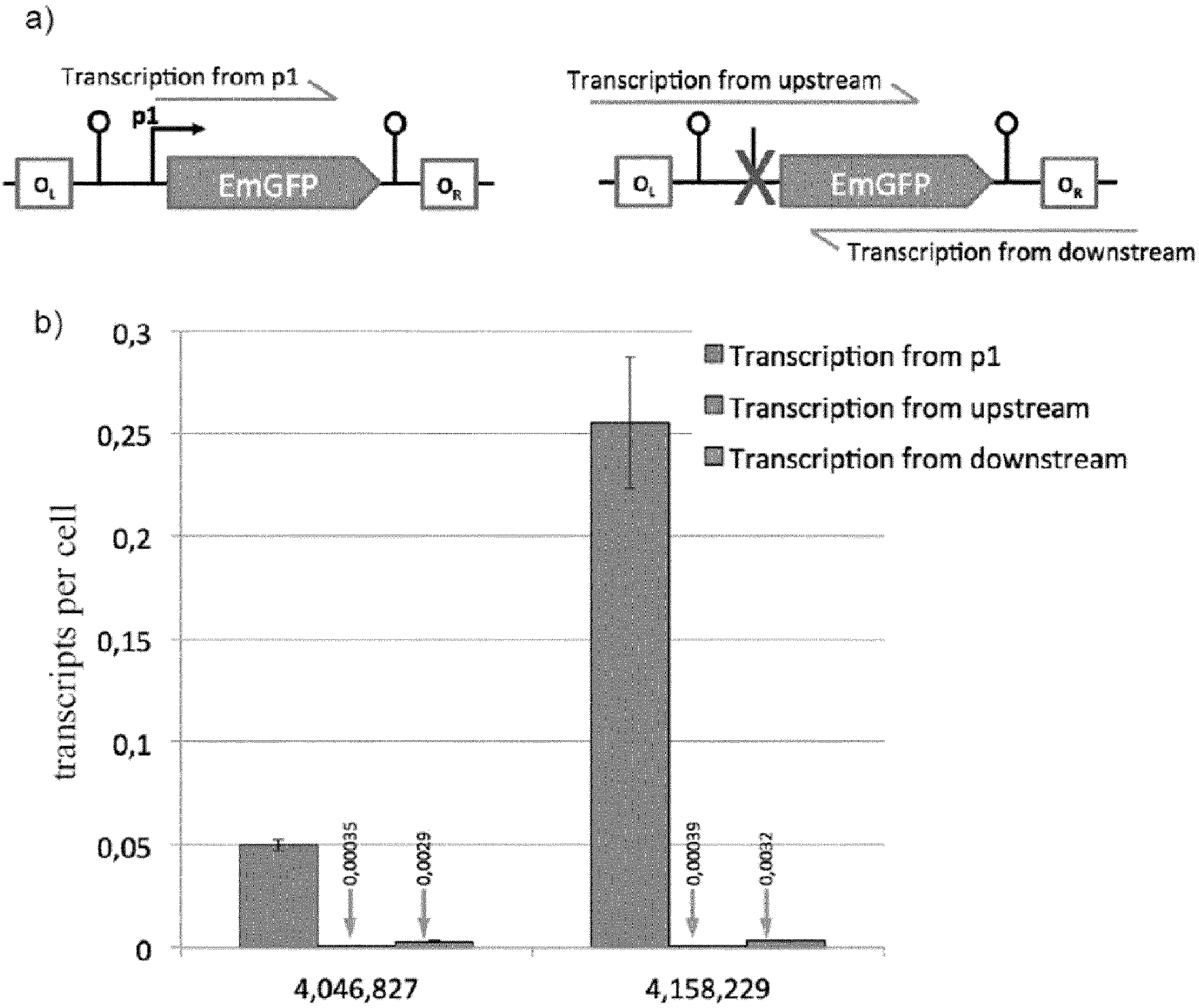
a)
Transcription from p1
p1
O_L
EmGFP
O_R
Transcription from upstream
O_L
EmGFP
O_R
Transcription from downstream
b)
transcripts per cell
0,3
0,25
0,2
0,15
0,1
0,05
0
0,00035
0,0029
0,00039
0,0032
4,046,827
4,158,229
Transcription from p1
Transcription from upstream
Transcription from downstream

**Figure 7**

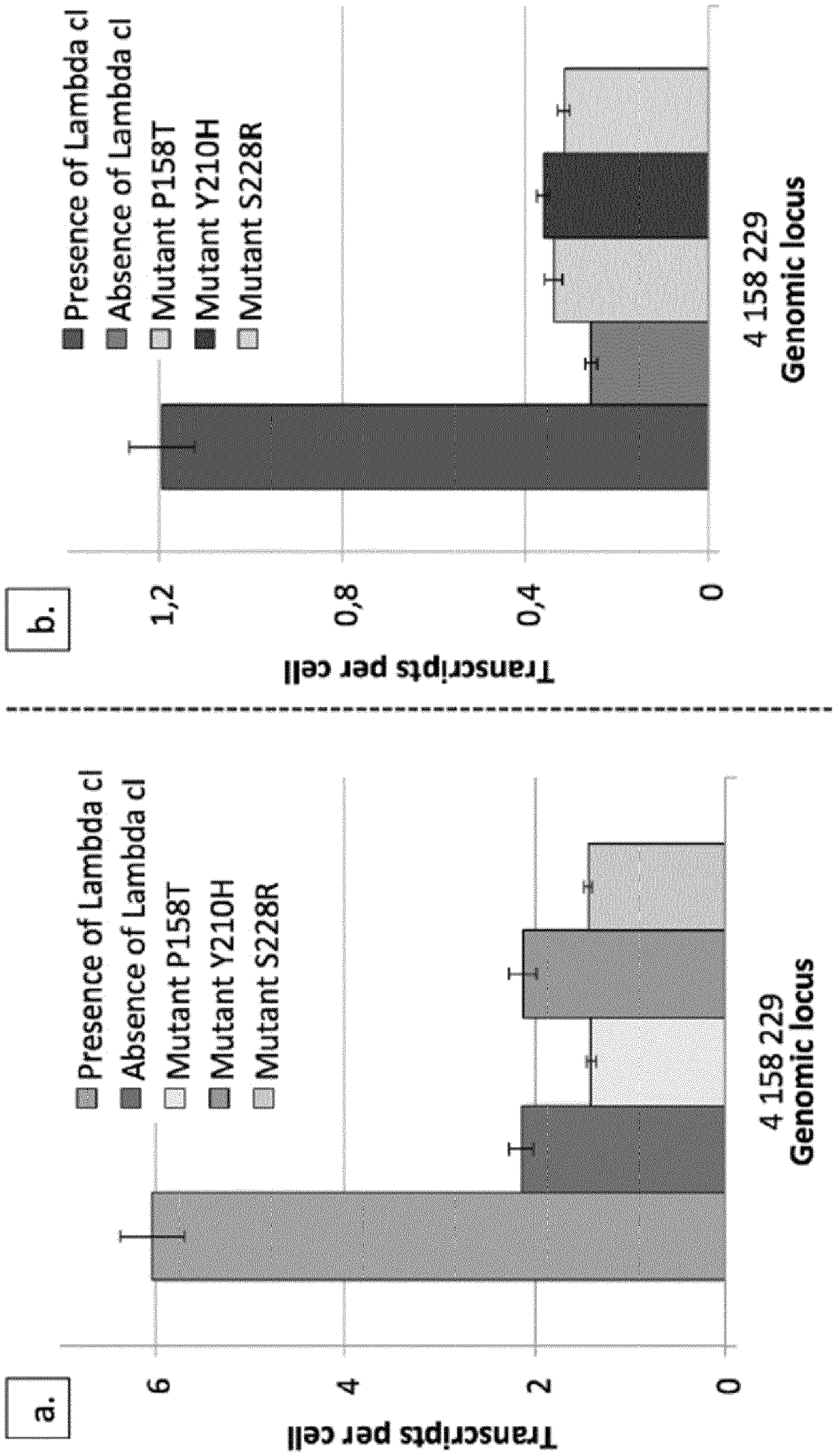
a.
Transcripts per cell
6
4
2
0
Presence of Lambda cI
Absence of Lambda cI
Mutant P158T
Mutant Y210H
Mutant S228R
4 158 229
Genomic locus
b.
Transcripts per cell
1,2
0,8
0,4
0
Presence of Lambda cI
Absence of Lambda cI
Mutant P158T
Mutant Y210H
Mutant S228R
4 158 229
Genomic locus

Figure 8

Europäisches Patentamt
European Patent Office
Office européen des brevets

# EUROPEAN SEARCH REPORT

Application Number
EP 20 20 9658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NAN HAO ET AL: "Programmable DNA looping using engineered bivalent dCas9 complexes", NATURE COMMUNICATIONS, vol. 8, no. 1, 20 November 2017 (2017-11-20), XP055713893, DOI: 10.1038/s41467-017-01873-x | 1,12-15 | INV. C12N15/74 C12N15/00 C12N15/79 C07K14/47 C12N15/63 |
| Y | * see abstract and pages 2-6 and Figures 1-2 and 4-5 * | 1-17 | |
| Y | MORGAN STEFANIE L. ET AL: "Manipulation of nuclear architecture through CRISPR-mediated chromosomal looping", NATURE COMMUNICATIONS, vol. 8, no. 1, 13 July 2017 (2017-07-13), XP055801808, DOI: 10.1038/ncomms15993 Retrieved from the Internet: URL:https://www.nature.com/articles/ncomms15993.pdf> * see abstract and pages 2-5, fig. 1-2 * | 1-17 | |
| | | | TECHNICAL FIELDS SEARCHED (IPC) |
| Y | COURNAC A. ET AL: "DNA Looping in Prokaryotes: Experimental and Theoretical Approaches", JOURNAL OF BACTERIOLOGY (PRINT), vol. 195, no. 6, 15 March 2013 (2013-03-15), pages 1109-1119, XP055801751, US ISSN: 0021-9193, DOI: 10.1128/JB.02038-12 Retrieved from the Internet: URL:https://jb.asm.org/content/jb/195/6/1109.full.pdf> * the whole document * | 1-17 | C12N C07K |

-/--

The present search report has been drawn up for all claims

3

| Place of search | Date of completion of the search | Examiner |
|---|---|---|
| Munich | 12 May 2021 | Vix, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 9658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PRIEST DAVID G. ET AL: "Quantitation of interactions between two DNA loops demonstrates loop domain insulation in E. coli cells", PROCEEDINGS OF THE NATIONAL ACADEMY OF SCIENCES, vol. 111, no. 42, 21 October 2014 (2014-10-21), pages E4449-E4457, XP055801754, US ISSN: 0027-8424, DOI: 10.1073/pnas.1410764111 Retrieved from the Internet: URL:https://www.pnas.org/content/pnas/111/42/E4449.full.pdf> * the whole document * ----- | 1-17 | |
| Y | HENSEL ZACH ET AL: "Transcription-Factor-Mediated DNA Looping Probed by High-Resolution, Single-Molecule Imaging in Live E. coli Cells", PLOS BIOLOGY, vol. 11, no. 6, 1 January 2013 (2013-01-01), page e1001591, XP055801756, DOI: 10.1371/journal.pbio.1001591 Retrieved from the Internet: URL:https://storage.googleapis.com/plos-corpus-prod/10.1371/journal.pbio.1001591/1/pbio.1001591.pdf?X-Goog-Algorithm=GOOG4-RSA-SHA256&X-Goog-Credential=wombat-sa@plos-prod.iam.gserviceaccount.com/20210505/auto/storage/goog4_request&X-Goog-Date=20210505T161647Z&X-Goog-Expires=3600&X-Goog-SignedHeaders=ho> * the whole document * ----- -/-- | 1-17 | TECHNICAL FIELDS SEARCHED (IPC) |

The present search report has been drawn up for all claims

3

| Place of search | Date of completion of the search | Examiner |
|---|---|---|
| Munich | 12 May 2021 | Vix, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 9658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | AYDIN AKBUDAK M. ET AL: "Effect of gene order in DNA constructs on gene expression upon integration into plant genome", 3 BIOTECH, vol. 7, no. 2, 29 May 2017 (2017-05-29), XP055801665, DE ISSN: 2190-572X, DOI: 10.1007/s13205-017-0729-2 Retrieved from the Internet: URL:https://link.springer.com/content/pdf/10.1007/s13205-017-0729-2.pdf> * the whole document * ----- | 1-17 | |
| Y | WO 2018/129544 A1 (WHITEHEAD INST BIOMEDICAL RES [US]) 12 July 2018 (2018-07-12) * see claims 1-49 * ----- | 1-17 | |
| Y,D | JACK A. BRYANT ET AL: "Chromosome position effects on gene expression in Escherichia coli K-12", NUCLEIC ACIDS RESEARCH, vol. 42, no. 18, 13 October 2014 (2014-10-13), pages 11383-11392, XP055626181, ISSN: 0305-1048, DOI: 10.1093/nar/gku828 * see abstract and pages 11385-11387 * ----- -/-- | 1-17 | TECHNICAL FIELDS SEARCHED (IPC) |

The present search report has been drawn up for all claims

3

| Place of search | Date of completion of the search | Examiner |
|---|---|---|
| Munich | 12 May 2021 | Vix, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 9658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | KIM JI HUN ET AL: "LADL: light-activated dynamic looping for endogenous gene expression control", NATURE METHODS, NATURE PUB. GROUP, NEW YORK, vol. 16, no. 7, 24 June 2019 (2019-06-24), pages 633-639, XP036901128, ISSN: 1548-7091, DOI: 10.1038/S41592-019-0436-5 [retrieved on 2019-06-24]<br>* see abstract, conlcusion and figures 1 and 5 *<br>----- | 1,12-15<br><br>1-17 | |
| A,D | CHONG SHASHA ET AL: "Mechanism of Transcriptional Bursting in Bacteria", CELL, ELSEVIER, AMSTERDAM NL, vol. 158, no. 2, 17 July 2014 (2014-07-17), pages 314-326, XP029036937, ISSN: 0092-8674, DOI: 10.1016/J.CELL.2014.05.038<br>* see abstract and pages 314-317 and Fig. 1 *<br>----- | 1-17 | TECHNICAL FIELDS SEARCHED (IPC) |
| A,D | SCOTT A SCHOLZ: "High-Resolution Mapping of the Escherichia coli Chromosome Reveals Positions of High and Low Transcription", CELL SYSTEMS, CELL PRESS, US , vol. 8, no. 3 27 March 2019 (2019-03-27), pages 212-225.e9, XP009516279, ISSN: 2405-4712, DOI: 10.1016/J.CELS.2019.02.004 Retrieved from the Internet: URL:https://doi.org/10.1016/j.cels.2019.02.004<br>* the whole document *<br>-----<br>-/-- | 1-17 | |

The present search report has been drawn up for all claims

| Place of search | Date of completion of the search | Examiner |
|---|---|---|
| Munich | 12 May 2021 | Vix, Olivier |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 20 9658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | JENNIFER DUMONT ET AL: "Human cell lines for biopharmaceutical manufacturing: history, status, and future perspectives", CRITICAL REVIEWS IN BIOTECHNOLOGY, vol. 36, no. 6, 18 September 2015 (2015-09-18), pages 1110-1122, XP055532666, US ISSN: 0738-8551, DOI: 10.3109/07388551.2015.1084266 * the whole document * ----- | 1-17 | |
| | | | TECHNICAL FIELDS SEARCHED (IPC) |

The present search report has been drawn up for all claims

3

| Place of search | Date of completion of the search | Examiner |
|---|---|---|
| Munich | 12 May 2021 | Vix, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 20 9658

This annex lists the patent family members relating to the patent documents cited in the above-mentioned European search report. The members are as contained in the European Patent Office EDP file on
The European Patent Office is in no way liable for these particulars which are merely given for the purpose of information.

12-05-2021

| Patent document cited in search report | Publication date | Patent family member(s) | Publication date |
|---|---|---|---|
| WO 2018129544 A1 | 12-07-2018 | EP 3565891 A1 | 13-11-2019 |
| | | WO 2018129544 A1 | 12-07-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **DUMONT et al.** *Crit Rev Biotechnol.,* 01 November 2016, vol. 36 (6), 1110-1122
- *Journal of Bacteriology,* 2013, vol. 195 (6), 1109-1119
- **BRYANT JA ; SELLARS LE ; BUSBY SJW ; LEE DJ.** Chromosome position effects on gene expression in Escherichia coli K-12. *Nucleic Acids Res.,* 2014, vol. 42, 11383-92
- **SCHOLZ SA ; DIAO R ; WOLFE MB ; FIVENSON EM ; LIN XN ; FREDDOLINO PL.** High-Resolution Mapping of the Escherichia coli Chromosome Reveals Positions of High and Low Transcription. *Cell Syst.,* 2019, vol. 8, 212-225
- **KOLKHOF P ; MÜLLER-HILL B.** Lambda cl Repressor Mutants Altered in Transcriptional Activation. *J Mol Biol.,* 1994, vol. 242, 23-36
- **YEUNG E ; DY AJ ; MARTIN KB ; NG AH ; DEL VECCHIO D ; BECK JL et al.** Biophysical Constraints Arising from Compositional Context in Synthetic Gene Networks. *Cell Syst.,* 2017, vol. 5, 11-24
- **PETER BJ ; ARSUAGA J ; BREIER AM ; KHODURSKY AB ; BROWN PO ; COZZARELLI NR.** Genomic transcriptional response to loss of chromosomal supercoiling in Escherichia coli. *Genome Biol.,* 2004, vol. 5, R87
- **POSTOW L ; HARDY CD ; ARSUAGA J ; COZZARELLI NR.** Topological domain structure of the Escherichia coli chromosome. *Genes Dev.,* 2004, vol. 18, 1766-79
- **DENG S ; STEIN RA ; HIGGINS NP.** Organization of supercoil domains and their reorganization by transcription. *Mol Microbiol.,* 2005, vol. 57, 1511-21
- **LIU LF ; WANG JC.** Supercoiling of the DNA template during transcription. *Proc Natl Acad Sci USA.,* 1987, vol. 84, 7024-7
- **MA J ; BAI L ; WANG MD.** Transcription under torsion. *Science,* 2013, vol. 340, 1580-3
- **MA J ; WANG M.** Interplay between DNA supercoiling and transcription elongation. *Transcription,* 2014, vol. 5, e28636
- **TANIGUCHI Y ; CHOI PJ ; LI G-W ; CHEN H ; BABU M ; HEARN J et al.** Quantifying E. coli proteome and transcriptome with single-molecule sensitivity in single cells. *Science,* 2010, vol. 329, 533-8
- **WAGNER R.** Transcription regulation in prokaryotes. Oxford University Press, 2000
- **CHONG S ; CHEN C ; GE H ; XIE XS.** Mechanism of Transcriptional Bursting in Bacteria. *Cell,* 2014, vol. 158, 314-26
- **EL HOUDAIGUI B ; FORQUET R ; HINDRÉ T ; SCHNEIDER D ; NASSER W ; REVERCHON S et al.** Bacterial genome architecture shapes global transcriptional regulation by DNA supercoiling. *Nucleic Acids Res.,* 2019, vol. 47, 5648-57
- **DORMAN CJ.** Genome architecture and global gene regulation in bacteria: making progress towards a unified model?. *Nat Rev Microbiol.,* 2013, vol. 11, 349-55
- **JIN DJ ; CAGLIERO C ; ZHOU YN.** Growth rate regulation in Escherichia coli. *FEMS Microbiol Rev.,* 2012, vol. 36, 269-87
- **JIN DJ ; CAGLIERO C ; MARTIN CM ; IZARD J ; ZHOU YN.** The dynamic nature and territory of transcriptional machinery in the bacterial chromosome. *Front Microbiol.,* 2015, vol. 6, 497
- **COURNAC A ; PLUMBRIDGE J.** DNA looping in prokaryotes: experimental and theoretical approaches. *J Bacteriol.,* 2013, vol. 195, 1109-19
- **MÜLLER-HILL B.** The lac Operon: a short history of a genetic paradigm. Walter de Gruyter, 1996
- **VILAR JMG ; LEIBLER S.** DNA looping and physical constraints on transcription regulation. *J Mol Biol.,* 2003, vol. 331, 981-9
- **VINOGRAD J ; LEBOWITZ J ; RADLOFF R ; WATSON R ; LAIPIS P.** The twisted circular form of polyoma viral DNA. *Proc Natl Acad Sci.,* 1965, vol. 53, 1104-11
- **STRICK TR ; ALLEMAND JF ; BENSIMON D ; BENSIMON A ; CROQUETTE V.** The elasticity of a single supercoiled DNA molecule. *Science,* 1996, vol. 271, 1835-7
- **LEPAGE T ; KÉPÈS F ; JUNIER I.** Thermodynamics of long supercoiled molecules: insights from highly efficient Monte Carlo simulations. *Biophys J.,* 2015, vol. 109, 135-43
- **KOUZINE F ; GUPTA A ; BARANELLO L ; WOJTOWICZ D ; BENAISSA K ; LIU J et al.** Transcription dependent dynamic supercoiling is a short-range genomic force. *Nat Struct Mol Biol.,* 2013, vol. 20, 396-403

- **PALMA CSD ; KANDAVALLI V ; BAHRUDEEN MNM ; MINOIA M ; CHAUHAN V ; DASH S et al.** Dissecting the in vivo dynamics of transcription locking due to positive supercoiling buildup. *Biochim Biophys Acta Gene Regul Mech.,* 2020, vol. 1863, 194515
- **WANG JC.** Moving one DNA double helix through another by a type II DNA topoisomerase: the story of a simple molecular machine. *Q Rev Biophys.,* 1998, vol. 31, 107-44
- **LAL A ; DHAR A ; TROSTEL A ; KOUZINE F ; SESHASAYEE ASN ; ADHYA S.** Genome scale patterns of supercoiling in a bacterial chromosome. *Nat Commun.,* 2016, vol. 7, 11055
- **CHAMPOUX JJ.** DNA Topoisomerases: Structure, Function, and Mechanism. *Annu Rev Biochem.,* 2001, vol. 70, 369-413
- **GELLERT M ; MIZUUCHI K ; O'DEA MH ; NASH HA.** DNA gyrase: an enzyme that introduces superhelical turns into DNA. *Proc Natl Acad Sci USA.,* 1976, vol. 73, 3872-6
- **ZECHIEDRICH EL ; KHODURSKY AB ; BACHELLIER S ; SCHNEIDER R ; CHEN D ; LILLEY DM et al.** Roles of topoisomerases in maintaining steady-state DNA supercoiling in Escherichia coli. *J Biol Chem.,* 2000, vol. 275, 8103-13
- **KHODURSKY AB ; PETER BJ ; SCHMID MB ; DERISI J ; BOTSTEIN D ; BROWN PO et al.** Analysis of topoisomerase function in bacterial replication fork movement: Use of DNA microarrays. *Proc Natl Acad Sci USA.,* 2000, vol. 97, 9419-24
- **SINDEN RR ; PETTIJOHN DE.** Chromosomes in living Escherichia coli cells are segregated into domains of supercoiling. *Proc Natl Acad Sci USA.,* 1981, vol. 78, 224-8
- **KAMAGATA K ; MANO E ; OUCHI K ; KANBAYASHI S ; JOHNSON RC.** High Free-Energy Barrier of 1D Diffusion Along DNA by Architectural DNA-Binding Proteins. *J Mol Biol.,* 2018, vol. 430, 655-67
- **DAGES S ; ZHI X ; LENG F.** Fis protein forms DNA topological barriers to confine transcription-coupled DNA supercoiling in Escherichia coli. *FEBS Lett.,* 2020, vol. 594, 791-8
- **JAPARIDZE A ; YANG W ; DEKKER C ; NASSER W ; MUSKHELISHVILI G.** DNA sequence-directed cooperation between nucleoid-associated proteins. preprint. *Biophysics,* 2020
- **HIGGINS NP ; YANG X ; FU Q ; ROTH JR.** Surveying a supercoil domain by using the gamma delta resolution system in Salmonella typhimurium. *J Bacteriol.,* 1996, vol. 178, 2825-35
- **YAN Y ; DING Y ; LENG F ; DUNLAP D ; FINZI L.** Protein-mediated loops in supercoiled DNA create large topological domains. *Nucleic Acids Res.,* 2018, vol. 46, 4417-24
- **LENG F ; CHEN B ; DUNLAP DD.** Dividing a supercoiled DNA molecule into two independent topological domains. *Proc Natl Acad Sci USA.,* 2011, vol. 108, 19973-8
- **MOULIN L ; RAHMOUNI AR ; BOCCARD F.** Topological insulators inhibit diffusion of transcription-induced positive supercoils in the chromosome of Escherichia coli: Diffusion of supercolis and topogical insulators. *Mol Microbiol.,* 2004, vol. 55, 601-10
- **DIMRI GP ; RUDD KE ; MORGAN MK ; BAYAT H ; AMES GF.** Physical mapping of repetitive extragenic palindromic sequences in Escherichia coli and phylogenetic distribution among Escherichia coli strains and other enteric bacteria. *J Bacteriol.,* 1992, vol. 174, 4583-93
- **VERMA SC ; QIAN Z ; ADHYA SL.** Architecture of the Escherichia coli nucleoid. *PLOS Genet.,* 2019, vol. 15, e1008456
- **REVET B ; WILCKEN-BERGMANN B VON ; BESSERT H ; BARKER A ; MÜLLER-HILL B.** Four dimers of λ repressor bound to two suitably spaced pairs of λ operators form octamers and DNA loops over large distances. *Curr Biol.,* 1999, vol. 9, 151-4
- **DODD IB ; PERKINS AJ ; TSEMITSIDIS D ; EGAN JB.** Octamerization of λ CI repressor is needed for effective repression of P RM and efficient switching from lysogeny. *Genes Dev.,* 2001, vol. 15, 3013-22
- **DODD IB ; SHEARWIN KE ; PERKINS AJ ; BURR T ; HOCHSCHILD A ; EGAN JB.** Cooperativity in long-range gene regulation by the lambda CI repressor. *Genes Dev.,* 2004, vol. 18, 344-54
- **DING Y ; MANZO C ; FULCRAND G ; LENG F ; DUNLAP D ; FINZI L.** DNA supercoiling: a regulatory signal for the λ repressor. *Proc Natl Acad Sci USA.,* 2014, vol. 111, 15402-7
- **ORAM M ; PATO ML.** Mu-like prophage strong gyrase site sequences: analysis of properties required for promoting efficient mu DNA replication. *J Bacteriol.,* 2004, vol. 186, 4575-84
- **BURZ DS ; ACKERS GK.** Single-site mutations in the C-terminal domain of bacteriophage lambda cl repressor alter cooperative interactions between dimers adjacently bound to OR. *Biochemistry,* 1994, vol. 33, 8406-16
- **KRONER GM ; WOLFE MB ; FREDDOLINO PL.** Escherichia coli Lrp Regulates One-Third of the Genome via Direct, Cooperative, and Indirect Routes. *J Bacteriol.,* 2019, 201
- **TANI TH ; KHODURSKY A ; BLUMENTHAL RM ; BROWN PO ; MATTHEWS RG.** Adaptation to famine: a family of stationary-phase genes revealed by microarray analysis. *Proc Natl Acad Sci USA.,* 2002, vol. 99, 13471-6

- **CHEN S ; HAO Z ; BIENIEK E ; CALVO JM.** Modulation of Lrp action in Escherichia coli by leucine: effects on non-specific binding of Lrp to DNA. *J Mol Biol.,* 2001, vol. 314, 1067-75
- **CHANDLER MG ; PRITCHARD RH.** The effect of gene concentration and relative gene dosage on gene output in Escherichia coli. *Mol Gen Genet MGG.,* 1975, vol. 138, 127-41
- **OEHLER S ; MÜLLER-HILL B.** High local concentration: a fundamental strategy of life. *J Mol Biol.,* 2010, vol. 395, 242-53
- **KÉPÈS F ; JESTER BC ; LEPAGE T ; RAFIEI N ; ROSU B ; JUNIER I.** The layout of a bacterial genome. *FEBS Lett.,* 2012, vol. 586, 2043-8
- **JUNIER I ; RIVOIRE O.** Conserved Units of Co-Expression in Bacterial Genomes: An Evolutionary Insight into Transcriptional Regulation. *PLoS ONE,* 2016, 11
- **JEONG KS ; AHN J ; KHODURSKY AB.** Spatial patterns of transcriptional activity in the chromosome of Escherichia coli. *Genome Biol.,* 2004, vol. 5, R86
- **TAPIAS A ; LOPEZ G ; AYORA S.** Bacillus subtilis LrpC is a sequence-independent DNA-binding and DNA-bending protein which bridges DNA. *Nucleic Acids Res.,* 2000, vol. 28, 552-9
- **DE LOS RIOS S ; PERONA JJ.** Structure of the Escherichia coli leucine-responsive regulatory protein Lrp reveals a novel octameric assembly. *J Mol Biol.,* 2007, vol. 366, 1589-602
- **PUL U ; LUX B ; WURM R ; WAGNER R.** Effect of upstream curvature and transcription factors H-NS and LRP on the efficiency of Escherichia coli rRNA promoters P1 and P2 - a phasing analysis. *Microbiol Read Engl.,* 2008, vol. 154, 2546-58
- **PUL U ; WURM R ; WAGNER R.** The role of LRP and H-NS in transcription regulation: involvement of synergism, allostery and macromolecular crowding. *J Mol Biol.,* 2007, vol. 366, 900-15
- **PUL U ; WURM R ; LUX B ; MELTZER M ; MENZEL A ; WAGNER R.** LRP and H-NScooperative partners for transcription regulation at Escherichia coli rRNA promoters. *Mol Microbiol.,* 2005, vol. 58, 864-76
- **GAAL T ; BRATTON BP ; SANCHEZ-VAZQUEZ P ; SLIWICKI A ; SLIWICKI K ; VEGEL A et al.** Colocalization of distant chromosomal loci in space in E. coli: a bacterial nucleolus. *Genes Dev.,* 2016, vol. 30, 2272-85
- **QIAN Z ; TROSTEL A ; LEWIS DEA ; LEE SJ ; HE X ; STRINGER AM et al.** Genome-Wide Transcriptional Regulation and Chromosome Structural Arrangement by GalR in E. coli. *Front Mol Biosci.,* 2016, vol. 3
- **YUS E ; LLORÉNS-RICO V ; MARTINEZ S ; GALLO C ; EILERS H ; BLÖTZ C et al.** Determination of the Gene Regulatory Network of a Genome-Reduced Bacterium Highlights Alternative Regulation Independent of Transcription Factors. *Cell Syst.,* 2019, vol. 9, 143-158
- **ROSSIGNOL M ; BASSET A ; ESPÉLI O ; BOCCARD F.** NKBOR, a mini-Tn10-based transposon for random insertion in the chromosome of Gram-negative bacteria and the rapid recovery of sequences flanking the insertion sites in Escherichia coli. *Res Microbiol.,* 2001, vol. 152, 481-5
- **KUHLMAN TE ; COX EC.** Site-specific chromosomal integration of large synthetic constructs. *Nucleic Acids Res.,* 2010, vol. 38, e92
- **KUHLMAN TE ; COX EC.** A place for everything: chromosomal integration of large constructs. *Bioeng Bugs,* 2010, vol. 1, 296-9
- **KESELER IM ; MACKIE A ; SANTOS-ZAVALETA A ; BILLINGTON R ; BONAVIDES-MARTÍNEZ C ; CASPI R et al.** The EcoCyc database: reflecting new knowledge about Escherichia coli K-12. *Nucleic Acids Res.,* 2017, (45), D543-50
- **GREEN MR ; SAMBROOK J.** Molecular Cloning: A Laboratory Manual. Cold Spring Harbor Laboratory Press, 2012
- Calculations: Converting from nanograms to copy number. *Default,* 23 June 2020, https://eu.idtdna.com/pages/education/decoded/article/calculations-converting-from-nanograms-to-copy-number
- **PHILIPS RM& R.** *How many mRNAs are in a cell?,* 23 June 2020, http://book.bionumbers.org/how-many-mrnas-are-in-a-cell

SEQUENCE LISTING

<110> SYNOVANCE

<120> METHODS FOR ENGINEERING CHROMOSOMAL ARCHITECTURE FOR GENE EXPRESSION

<130> BRV 185 - EP PRIO 2

<160> 99

<170> PatentIn version 3.5

<210> 1
<211> 61
<212> DNA
<213> Artificial Sequence

<220>
<223> lambda CI associated sequence

<400> 1
tatcaccgcc agtggtattt atgtcaacac cgccagagat aatttatcac cgcagatggt 60

t 61

<210> 2
<211> 64
<212> DNA
<213> Artificial Sequence

<220>
<223> lambda CI associated sequence

<400> 2
tatcaccgca agggataaat atctaacacc gtgcgtgttg actattttac ctctggcggt 60

gata 64

<210> 3
<211> 70
<212> DNA
<213> Artificial Sequence

<220>
<223> Binds pBCJ927/pBCJ932 targeting location 2 171 769 on genome

<400> 3
tgaattcatc gtcatttacc catattcaat tgtggctagt gtaaacgaag tacggcccca 60

aggtccaaac 70

<210> 4
<211> 70
<212> DNA
<213> Artificial Sequence

<220>
<223> Binds pBCJ927/pBCJ932 targeting location 2 171 769 on genome

<400> 4
gtgatgctgg cccggtattg tgcaaaacag atcattcacc aatggtcccc ttggcttcag 60

ggatgaggcg 70

<210> 5
<211> 70
<212> DNA
<213> Artificial Sequence

<220>
<223> Binds pBCJ927/pBCJ932 targeting location 2 185 490 on genome

<400> 5
gataaatcgc agaggaggat ggtaatgtcc agcgcacgcg ttgtaaacga tacggcccca 60

aggtccaaac 70

<210> 6
<211> 70
<212> DNA
<213> Artificial Sequence

<220>
<223> Binds pBCJ927/pBCJ932 targeting location 2 185 490 on genome

<400> 6
ttcatatatc aaataattta ttaacgcgat tgtaaaactg ccgtttttcc ttggcttcag 60

ggatgaggcg 70

<210> 7
<211> 70
<212> DNA
<213> Artificial Sequence

<220>
<223> Binds pBCJ927/pBCJ932 targeting location 2 192 420 on genome

<400> 7
aatatttaag agtattaact atttatcgca tctatcaatt aatgtagatt tacggcccca 60

aggtccaaac 70

<210> 8
<211> 70
<212> DNA
<213> Artificial Sequence

<220>
<223> Binds pBCJ927/pBCJ932 targeting location 2 192 420 on genome

<400> 8
atataaatga tttcggcttt tttattgata tcaacaatac catttacata ttggcttcag 60

ggatgaggcg 70

<210> 9

<211> 70
<212> DNA
<213> Artificial Sequence

<220>
<223> Binds pBCJ927/pBCJ932 targeting location 2 196 391 on genome

<400> 9
catcgacagc gccttttctt tataaattcc taaagttgtt ttcttgcgat tacggcccca 60

aggtccaaac 70

<210> 10
<211> 70
<212> DNA
<213> Artificial Sequence

<220>
<223> Binds pBCJ927/pBCJ932 targeting location 2 196 391 on genome

<400> 10
cagttgaatg cagatgctac cagtatttat gcgggttaga gagagacaaa ttggcttcag 60

ggatgaggcg 70

<210> 11
<211> 70
<212> DNA
<213> Artificial Sequence

<220>
<223> Binds pBCJ927/pBCJ932 targeting location 2 214 800 on genome

<400> 11
aaatcgcctg gcaaaaataa aatcacccta tagatgcaca aaaaacgggc tacggcccca 60

aggtccaaac 70

<210> 12
<211> 70
<212> DNA
<213> Artificial Sequence

<220>
<223> Binds pBCJ927/pBCJ932 targeting location 2 214 800 on genome

<400> 12
gcgaggtccc ggtttaactt tagacgcagt tttgcgaacc aggtagtttt ttggcttcag 60

ggatgaggcg 70

<210> 13
<211> 70
<212> DNA
<213> Artificial Sequence

<220>
<223> Binds pBCJ927/pBCJ932 targeting location 2 223 904 on genome

<400> 13
ggatcggtaa aaccagtaaa cggaaaaact ggcaggaagt ggagtaaaaa tacggcccca 60

aggtccaaac 70

<210> 14
<211> 70
<212> DNA
<213> Artificial Sequence

<220>
<223> Binds pBCJ927/pBCJ932 targeting location 2 223 904 on genome

<400> 14
ggcacagaac gattaagtga attcggatgg cgatactctg ccatccgtaa ttggcttcag 60

ggatgaggcg 70

<210> 15
<211> 70
<212> DNA
<213> Artificial Sequence

<220>
<223> Binds pBCJ927/pBCJ932 targeting location 2 244 773 on genome

<400> 15
catgcgcagt atttactgaa gtgaaagtcc gcccggttcg ccgggcatct tacggcccca 60

aggtccaaac 70

<210> 16
<211> 70
<212> DNA
<213> Artificial Sequence

<220>
<223> Binds pBCJ927/pBCJ932 targeting location 2 244 773 on genome

<400> 16
gaagacggac gtcgttactt tatggcagtg gattatcgct tctgatgaga ttggcttcag 60

ggatgaggcg 70

<210> 17
<211> 70
<212> DNA
<213> Artificial Sequence

<220>
<223> Binds pBCJ927/pBCJ932 targeting location 2 248 654 on genome

<400> 17
acacggttat aagacacctt catgatcgcc cagggattat aagtaaagca tacggcccca 60

aggtccaaac 70

<210> 18

<211> 70
<212> DNA
<213> Artificial Sequence

<220>
<223> Binds pBCJ927/pBCJ932 targeting location 2 248 654 on genome

<400> 18
attttcgtgac gcagcgcctt cagcatgcat tcgccagaaa agagattggc ttggcttcag 60

ggatgaggcg 70

<210> 19
<211> 80
<212> DNA
<213> Artificial Sequence

<220>
<223> Binds pBCJ927/pBCJ932 targeting location 3 959 452 on genome

<400> 19
gaaactgcgc ggctatatga cagatatgaa acgtattgct gttgcgggtt aagtgcgcgc 60

tacggcccca aggtccaaac 80

<210> 20
<211> 82
<212> DNA
<213> Artificial Sequence

<220>
<223> Binds pBCJ927/pBCJ932 targeting location 3 959 452 on genome

<400> 20
aggagaaggc cttgagtgtt ttctccctct ccctgtggga gagggtcggg gtgagggcat 60

cattggcttc agggatgagg cg 82

<210> 21
<211> 72
<212> DNA
<213> Artificial Sequence

<220>
<223> Binds pBCJ927/pBCJ932 targeting location 3 990 959 on genome

<400> 21
aataaggaat ttacagagaa taaacggtgc tacacttgta tgtagcgcat cttacggccc 60

caaggtccaa ac 72

<210> 22
<211> 70
<212> DNA
<213> Artificial Sequence

<220>
<223> Binds pBCJ927/pBCJ932 targeting location 3 990 959 on genome

<400> 22
atggtctaaa acgtgatcaa tttaacacct tgctgattga ccgtaaagaa ttggcttcag 60

ggatgaggcg 70

<210> 23
<211> 80
<212> DNA
<213> Artificial Sequence

<220>
<223> Binds pBCJ927/pBCJ932 targeting location 4 035 211 on genome

<400> 23
aagcataaag aataaaaaat gcgcggtcag aaaattattt taaatttcct cttgtcaggc 60

tacggcccca aggtccaaac 80

<210> 24
<211> 80
<212> DNA
<213> Artificial Sequence

<220>
<223> Binds pBCJ927/pBCJ932 targeting location 4 035 211 on genome

<400> 24
ccggcggcgt gtttgccgtt gttccgtgtc agtggtggcg cattataggg agttattccg 60

ttggcttcag ggatgaggcg 80

<210> 25
<211> 80
<212> DNA
<213> Artificial Sequence

<220>
<223> Binds pBCJ927/pBCJ932 targeting location 4 046 827 on genome

<400> 25
acaaacatca tgctgtaaaa agcatgataa taaattaaaa gcgatgtaaa taatttatgc 60

tacggcccca aggtccaaac 80

<210> 26
<211> 81
<212> DNA
<213> Artificial Sequence

<220>
<223> Binds pBCJ927/pBCJ932 targeting location 4 046 827 on genome

<400> 26
tcgtgaaaat cttttgtaga tcttctggat cgctcgcaaa tcgtcatgtg gataactttg 60

tttggcttca gggatgaggc g 81

<210> 27

<211> 80
<212> DNA
<213> Artificial Sequence

<220>
<223> Binds pBCJ927/pBCJ932 targeting location 4 158 229 on genome

<400> 27
tcaccgcagc aggtggcgca ggcgattgct tttgcgcagg ctcggttagg gtaagaacat 60

tacggcccca aggtccaaac 80

<210> 28
<211> 80
<212> DNA
<213> Artificial Sequence

<220>
<223> Binds pBCJ927/pBCJ932 targeting location 4 158 229 on genome

<400> 28
ccgctttttt ttgccataaa aaagcccggc gataagccag gctcaaattt atacatataa 60

ttggcttcag ggatgaggcg 80

<210> 29
<211> 84
<212> DNA
<213> Artificial Sequence

<220>
<223> Binds pBCJ927/pBCJ932 targeting location 4 166 287 on genome

<400> 29
tattgcccgt tttacagcgt tacggcttcg aaacgctcga aaaactggca gttttaggct 60

gatttacggc cccaaggtcc aaac 84

<210> 30
<211> 82
<212> DNA
<213> Artificial Sequence

<220>
<223> Binds pBCJ927/pBCJ932 targeting location 4 166 287 on genome

<400> 30
aaataatttt ctgaccgcgc aacattcaac caaatcagcc taaaactgcc agtttttcga 60

gcttggcttc agggatgagg cg 82

<210> 31
<211> 80
<212> DNA
<213> Artificial Sequence

<220>
<223> Binds pBCJ927/pBCJ932 targeting location 4 207 682 on genome

<400> 31
ttttcgcccg cattgtaacg aaaacgtttg cgcaacgctc gcgaattttt ctctttcaat 60

tacggcccca aggtccaaac 80

<210> 32
<211> 80
<212> DNA
<213> Artificial Sequence

<220>
<223> Binds pBCJ927/pBCJ932 targeting location 4 207 682 on genome

<400> 32
aatcatcaat gtaatttctg tattttgccc acggtaacca cagtcaaaat tgtgatcacc 60

ttggcttcag ggatgaggcg 80

<210> 33
<211> 80
<212> DNA
<213> Artificial Sequence

<220>
<223> Binds pBCJ927/pBCJ932 targeting location 4 207 682 on genome

<400> 33
tctgtggtat ccgctcatgt ttcgcgcggc gctacgcaaa cccgaatcat cggatttaac 60

tacggcccca aggtccaaac 80

<210> 34
<211> 80
<212> DNA
<213> Artificial Sequence

<220>
<223> Binds pBCJ927/pBCJ932 targeting location 4 207 682 on genome

<400> 34
gtggtattat tggccattga aagaaccttt ttacattatg agcgtcaata tcagtgtacc 60

ttggcttcag ggatgaggcg 80

<210> 35
<211> 20
<212> DNA
<213> Artificial Sequence

<220>
<223> Genomic control of insertion of Construction pBCJ927/pBCJ932

<400> 35
tactcccgcc agatcctgaa 20

<210> 36
<211> 20
<212> DNA

<213> Artificial Sequence

<220>
<223> Genomic control of insertion of Construction pBCJ927/pBCJ932

<400> 36
agccattaat cgctcaccgt 20

<210> 37
<211> 20
<212> DNA
<213> Artificial Sequence

<220>
<223> Genomic control of insertion of Construction pBCJ927/pBCJ932

<400> 37
cgagtcgttt ggttgcgatg 20

<210> 38
<211> 20
<212> DNA
<213> Artificial Sequence

<220>
<223> Genomic control of insertion of Construction pBCJ927/pBCJ932

<400> 38
aacacatcgt tcaccagggg 20

<210> 39
<211> 20
<212> DNA
<213> Artificial Sequence

<220>
<223> Genomic control of insertion of Construction pBCJ927/pBCJ932

<400> 39
gcggcagcaa taattgaacg 20

<210> 40
<211> 21
<212> DNA
<213> Artificial Sequence

<220>
<223> Genomic control of insertion of Construction pBCJ927/pBCJ932

<400> 40
gaagtggttg ttgcaggtag c 21

<210> 41
<211> 20
<212> DNA
<213> Artificial Sequence

<220>

<223> Genomic control of insertion of Construction pBCJ927/pBCJ932

<400> 41
ataatccccc ttcaaggcgc 20

<210> 42
<211> 20
<212> DNA
<213> Artificial Sequence

<220>
<223> Genomic control of insertion of Construction pBCJ927/pBCJ932

<400> 42
gcaactcgtt tccctgttgc 20

<210> 43
<211> 20
<212> DNA
<213> Artificial Sequence

<220>
<223> Genomic control of insertion of Construction pBCJ927/pBCJ932

<400> 43
tacccgcaaa ccacacttcc 20

<210> 44
<211> 20
<212> DNA
<213> Artificial Sequence

<220>
<223> Genomic control of insertion of Construction pBCJ927/pBCJ932

<400> 44
caaagcaacg ccacttcacc 20

<210> 45
<211> 20
<212> DNA
<213> Artificial Sequence

<220>
<223> Genomic control of insertion of Construction pBCJ927/pBCJ932

<400> 45
cgaccaacag catgaatccg 20

<210> 46
<211> 20
<212> DNA
<213> Artificial Sequence

<220>
<223> Genomic control of insertion of Construction pBCJ927/pBCJ932

<400> 46

acaaaaagcc gcccaaatgg 20

<210> 47
<211> 20
<212> DNA
<213> Artificial Sequence

<220>
<223> Genomic control of insertion of Construction pBCJ927/pBCJ932

<400> 47
gatgtatgcc agtaacgcgc 20

<210> 48
<211> 20
<212> DNA
<213> Artificial Sequence

<220>
<223> Genomic control of insertion of Construction pBCJ927/pBCJ932

<400> 48
actataccgg gcagaaacgc 20

<210> 49
<211> 20
<212> DNA
<213> Artificial Sequence

<220>
<223> Genomic control of insertion of Construction pBCJ927/pBCJ932

<400> 49
aaatgaggcg cgattgtagc 20

<210> 50
<211> 20
<212> DNA
<213> Artificial Sequence

<220>
<223> Genomic control of insertion of Construction pBCJ927/pBCJ932

<400> 50
ggcgctttct ggactattgc 20

<210> 51
<211> 20
<212> DNA
<213> Artificial Sequence

<220>
<223> Genomic control of insertion of Construction pBCJ927/pBCJ932

<400> 51
cttgtgtgcc gttgctgaaa 20

<210> 52
<211> 20
<212> DNA
<213> Artificial Sequence

<220>
<223> Genomic control of insertion of Construction pBCJ927/pBCJ932

<400> 52
tggttccggc gttcgataaa 20

<210> 53
<211> 20
<212> DNA
<213> Artificial Sequence

<220>
<223> Genomic control of insertion of Construction pBCJ927/pBCJ932

<400> 53
tggatcctga caggcgtttc 20

<210> 54
<211> 20
<212> DNA
<213> Artificial Sequence

<220>
<223> Genomic control of insertion of Construction pBCJ927/pBCJ932

<400> 54
ttggaatgca ggccccatag 20

<210> 55
<211> 20
<212> DNA
<213> Artificial Sequence

<220>
<223> Genomic control of insertion of Construction pBCJ927/pBCJ932

<400> 55
gtcaggcggt gaaacggata 20

<210> 56
<211> 20
<212> DNA
<213> Artificial Sequence

<220>
<223> Genomic control of insertion of Construction pBCJ927/pBCJ932

<400> 56
tttgctttct ctgccggagt 20

<210> 57
<211> 20
<212> DNA

<213> Artificial Sequence

<220>
<223> Genomic control of insertion of Construction pBCJ927/pBCJ932

<400> 57
gcaaggcgca gattttagca 20

<210> 58
<211> 20
<212> DNA
<213> Artificial Sequence

<220>
<223> Genomic control of insertion of Construction pBCJ927/pBCJ932

<400> 58
gatcttctgg atcgctcgca 20

<210> 59
<211> 20
<212> DNA
<213> Artificial Sequence

<220>
<223> Genomic control of insertion of Construction pBCJ927/pBCJ932

<400> 59
cgattctgtc gctgcaatcg 20

<210> 60
<211> 20
<212> DNA
<213> Artificial Sequence

<220>
<223> Genomic control of insertion of Construction pBCJ927/pBCJ932

<400> 60
actgcccgtt tcgagagttt 20

<210> 61
<211> 20
<212> DNA
<213> Artificial Sequence

<220>
<223> Genomic control of insertion of Construction pBCJ927/pBCJ932

<400> 61
ttacaagtgc tgccagaggg 20

<210> 62
<211> 20
<212> DNA
<213> Artificial Sequence

<220>

<223> Genomic control of insertion of Construction pBCJ927/pBCJ932

<400> 62
ccggcctgac aagaggaaat 20

<210> 63
<211> 20
<212> DNA
<213> Artificial Sequence

<220>
<223> Genomic control of insertion of Construction pBCJ927/pBCJ932

<400> 63
ttcgacgata ccggctttgt 20

<210> 64
<211> 20
<212> DNA
<213> Artificial Sequence

<220>
<223> Genomic control of insertion of Construction pBCJ927/pBCJ932

<400> 64
tgactgactg ctgcattccc 20

<210> 65
<211> 20
<212> DNA
<213> Artificial Sequence

<220>
<223> Genomic control of insertion of Construction pBCJ927/pBCJ932

<400> 65
agaggtatcc agccccagtt 20

<210> 66
<211> 20
<212> DNA
<213> Artificial Sequence

<220>
<223> Genomic control of insertion of Construction pBCJ927/pBCJ932

<400> 66
cttcgttgat ccatgcagcg 20

<210> 67
<211> 20
<212> DNA
<213> Artificial Sequence

<220>
<223> Lambda-cI genome insertion control

<400> 67

gagagatggg taagcacaac 20

<210> 68
<211> 20
<212> DNA
<213> Artificial Sequence

<220>
<223> Lambda-cI genome insertion control

<400> 68
tcaggtggct catcacgcta 20

<210> 69
<211> 20
<212> DNA
<213> Artificial Sequence

<220>
<223> EmGFP qPCR probes

<400> 69
tcgtgaccac cttgacctac 20

<210> 70
<211> 20
<212> DNA
<213> Artificial Sequence

<220>
<223> EmGFP qPCR probes

<400> 70
gtcgtccttg aagaagatgg 20

<210> 71
<211> 102
<212> DNA
<213> Artificial Sequence

<220>
<223> Overhangs containing lambda-Cioperator boxes

<400> 71
ggatacacat cttgtcatat gtatcaccgc cagtggtatt tatgtcaaca ccgccagaga 60

taatttatca ccgcagatgg tttacggccc caaggtccaa ac 102

<210> 72
<211> 105
<212> DNA
<213> Artificial Sequence

<220>
<223> Overhangs containing lambda-Cioperator boxes

<400> 72
ggatacacat cttgtcatat gtatcaccgc cagaggtaaa atagtcaaca cgcacggtgt 60

tagatattta tcccttgcgg tgatattggc ttcagggatg aggcg 105

<210> 73
<211> 49
<212> DNA
<213> Artificial Sequence

<220>
<223> Overhangs containing strong promoter p3 for pBCJ927

<400> 73
tcacccgggt cgtataatgt gtggatacta gtgaaagagg agaaatact 49

<210> 74
<211> 57
<212> DNA
<213> Artificial Sequence

<220>
<223> Overhangs containing strong promoter p3 for pBCJ927

<400> 74
cgacccgggt gaattaataa gcaaataaat tgtcgctatt gagtgagcta actcaca 57

<210> 75
<211> 76
<212> DNA
<213> Artificial Sequence

<220>
<223> Insertion of Lambda-CI in Rha operon

<400> 75
attcaggcgc tttttagact ggtcgtaatg aaattcagca ggatcaccat atgagcacaa 60

aaaagaaacc attaac 76

<210> 76
<211> 59
<212> DNA
<213> Artificial Sequence

<220>
<223> Insertion of Lambda-CI in Rha operon

<400> 76
gctcctttgt ctgtcgtgta gagtgaatct gcgccacatt tggcttcagg gatgaggcg 59

<210> 77
<211> 21
<212> DNA
<213> Artificial Sequence

<220>
<223> Insertion of GRS downstream emGFP

<400> 77

gcttgatatc gaattccgaa g 21

<210> 78
<211> 20
<212> DNA
<213> Artificial Sequence

<220>
<223> Insertion of GRS downstream emGFP

<400> 78
cttgtacagc tcgtccatgc 20

<210> 79
<211> 29
<212> DNA
<213> Artificial Sequence

<220>
<223> Insertion of GRS upstream emGFP

<400> 79
ggccgattca ttaatgcagc tggcacgac 29

<210> 80
<211> 29
<212> DNA
<213> Artificial Sequence

<220>
<223> Insertion of GRS upstream emGFP

<400> 80
aacgcgcggg gagaggcggt ttgcgtatt 29

<210> 81
<211> 27
<212> DNA
<213> Artificial Sequence

<220>
<223> Insertion of GRS downstream the loop

<400> 81
tagcttggct gcaggtcgtc gaaattc 27

<210> 82
<211> 20
<212> DNA
<213> Artificial Sequence

<220>
<223> Insertion of GRS downstream the loop

<400> 82
gcttggctgg acgtaaactc 20

<210>  83
<211>  66
<212>  DNA
<213>  Artificial Sequence

<220>
<223>  Insertion neutral DNA downstream the loop

<400>  83
catggtcata gctgtttcca gcgccggaag ttgtgtaaca gtcatgcccg gcgttctggt        60

ccattc                                                                   66

<210>  84
<211>  63
<212>  DNA
<213>  Artificial Sequence

<220>
<223>  Insertion neutral DNA downstream the loop

<400>  84
actggccgtc gttttacaca ccaggtttac ctatcgttct gattgcatat ccggtaactg        60

cgg                                                                      63

<210>  85
<211>  21
<212>  DNA
<213>  Artificial Sequence

<220>
<223>  Binds on Landing pad 1

<400>  85
tacggcccca aggtccaaac g                                                  21

<210>  86
<211>  20
<212>  DNA
<213>  Artificial Sequence

<220>
<223>  Binds on Landing pad 2

<400>  86
ttggcttcag ggatgaggcg                                                    20

<210>  87
<211>  64
<212>  DNA
<213>  Artificial Sequence

<220>
<223>  For mutation of p3 promoter

<400>  87
gcatccgggc gtgcgctcta tactagtgaa agaggagaaa tactagatgg taagcaaggg        60

cgag 64

<210> 88
<211> 91
<212> DNA
<213> Artificial Sequence

<220>
<223> For mutation of p3 promoter

<400> 88
tagagcgcac gcccggatgc gatgaatgtg ctatggacca taaggtcgct acgggacctg 60

ctaacaacgt tagagcctgt aactgcgcct g 91

<210> 89
<211> 114
<212> DNA
<213> Artificial Sequence

<220>
<223> mCherry from plasmid pMC48 with mutated promoter

<400> 89
tgtaactcac tcaatagcga cgcgagttgt aacgctccag ctgctagtag atagacatag 60

ctctaggcgt actagtgaaa gaggagaaat actagatggt gagcaagggc gagg 114

<210> 90
<211> 118
<212> DNA
<213> Artificial Sequence

<220>
<223> mCherry from plasmid pMC48 with mutated promoter

<400> 90
gcatcttcgg cattttgcc ccatgcaaac gggccgtggg aatggaccag aacgccgggc 60

aataaaaaag cccccggaat gatcttccgg gggcttactt gtacagctcg tccatgcc 118

<210> 91
<211> 26
<212> DNA
<213> Artificial Sequence

<220>
<223> pBCJ927 for integration of mutated promoter mCherry

<400> 91
ctcgcgtcgc tattgagtga gttaca 26

<210> 92
<211> 20
<212> DNA
<213> Artificial Sequence

<220>

<223> pBCJ927 for integration of mutated promoter mCherry

<400> 92
cgcctcatcc ctgaagccaa 20

<210> 93
<211> 20
<212> DNA
<213> Artificial Sequence

<220>
<223> mCherry qPCR probes

<400> 93
gaacggccac gagttcgaga 20

<210> 94
<211> 24
<212> DNA
<213> Artificial Sequence

<220>
<223> mCherry qPCR probes

<400> 94
cttggagccg tacatgaact gagg 24

<210> 95
<211> 44
<212> DNA
<213> Artificial Sequence

<220>
<223> Insertion of S228R mutation in Lambda-cI

<400> 95
gtggggaaag ttatcgctcg ccagtggcct gaagagacgt ttgg 44

<210> 96
<211> 46
<212> DNA
<213> Artificial Sequence

<220>
<223> Insertion of Y210H mutation in Lambda-cI

<400> 96
cactaaaccc acagcatcca atgatcccat gcaatgagag ttgttc 46

<210> 97
<211> 111
<212> DNA
<213> Artificial Sequence

<220>
<223> Insertion of P158T mutation in Lambda-cI

<400> 97

```
tgaggttgaa ggtaattcca tgaccgcacc aacaggctcc aagatcccgc cgaaaggcgg        60

gattttctta gggataacag ggtaatagtt gacaattaat catcggcata g                111


<210>  98
<211>  23
<212>  DNA
<213>  Artificial Sequence

<220>
<223>  Amplification of GRS

<400>  98
cccagtcacg acgttgtaaa acg                                                23


<210>  99
<211>  23
<212>  DNA
<213>  Artificial Sequence

<220>
<223>  Amplification of GRS

<400>  99
agcggataac aatttcacac agg                                                23
```